Europäisches Patentamt
European Patent Office
Office européen des brevets

(19)

(11) Veröffentlichungsnummer: **0 236 913**
**A2**

(12)

# EUROPÄISCHE PATENTANMELDUNG

(21) Anmeldenummer: **87103003.7**

(22) Anmeldetag: **03.03.87**

(51) Int.Cl.³: **C 07 D 233/56**
C 07 D 233/60, C 07 D 249/08
C 07 D 405/06, C 07 D 409/06
C 07 D 409/14, A 61 K 31/415
A 61 K 31/41, A 01 N 43/50
A 01 N 43/653

(30) Priorität: **12.03.86 DE 3608144**

(43) Veröffentlichungstag der Anmeldung:
**16.09.87 Patentblatt 87/38**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schaper, Wolfgang, Dr.**
**Rauenthaler Weg 18**
**D-6000 Frankfurt am Main(DE)**

(72) Erfinder: **Fuss, Andreas, Dr.**
**Lindigstrasse 24**
**D-8757 Karlstein(DE)**

(72) Erfinder: **Raether, Wolfgang, Dr.**
**Falkensteinstrasse 6**
**D-6072 Dreieich(DE)**

(72) Erfinder: **Dittmar, Walter, Dr.**
**Uhlandstrasse 10**
**D-6238 Hofheim am Taunus(DE)**

(72) Erfinder: **Hänel, Heinz, Dr.**
**Tannenwaldallee 80**
**D-6380 Bad Homburg(DE)**

(72) Erfinder: **Gerhards, Hermann Josef, Dr.**
**Wacholderweg 4**
**D-6238 Hofheim am Taunus(DE)**

(72) Erfinder: **Sachse, Burkhard, Dr.**
**An der Ziegelei 30**
**D-6233 Kelkheim (Taunus)(DE)**

(54) **Aryl-azolylmethyl-benzocycloalken-Derivate, Verfahren zu ihrer Herstellung und ihre Verwendung.**

(57) Azolverbindungen der Formel (I)

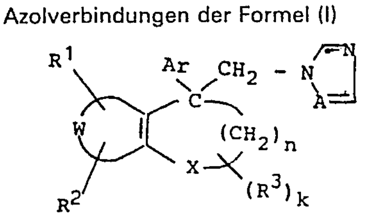

(I)

worin A = CH oder N, n = 1, 2 oder 3, k = 1 oder 2, Ar = ein (subst.) aromatischer Rest oder (subst.) Thienyl, W = die zur Vervollständigung eines Benzol-, Naphthalinoder Thiophen-Ringsystems nötigen Atome bedeutet, X = ein Rest $C(R^3)_2$ sowie für den Fall, daß W die zur Vervollständigung eines Benzol- oder Naphthalin-Ringsystems nötigen Atome bedeutet, auch Sauerstoff oder Schwefel, $R^1$ und $R^2$ = H, Halogen, (subst.) Alkyl, Cycloalkyl, Alkoxy, Alkylthio, Cyano oder Nitro bedeutet, sowie für den Fall, daß gleichzeitig W die zur Vervollständigung eines Benzolrings nötigen Atome bedeutet, $R^1$ auch Phenyl, Phenoxy, Phenylthio, Phenylsulfinyl, Phenylsulfonyl, Benzyl, Benzyloxy oder Hydroxy bedeutet, wobei die Phenylgruppen substituiert sein können oder $R^1$, $R^2$ zusammen, sofernsie o-ständig orientiert sind, eine Alkylenkette; $R^3$ = H oder Alkyl oder zwei Reste $R^3$ zusammen, wenn sie an dasselbe C-Atom gebunden sind, eine Alkylenkette sowie ihre Stereoisomeren und Salze besitzen vorteilhafte fungizide antimykotische und antikonvulsive Wirkungen.

EP 0 236 913 A2

Croydon Printing Company Ltd

HOECHST AKTIENGESELLSCHAFT HOE 86/F 054 Dr.AU/St

Aryl-azolylmethyl-benzocycloalken-Derivate, Verfahren zu ihrer Herstellung und ihre Verwendung

Die Erfindung betrifft 1-Aryl-1-azolylmethyl-benzocycloalken-Derivate, Verfahren zu ihrer Herstellung und ihre Verwendung als Pflanzenschutzmittel, insbesondere als Fungizide oder als Wachstumsregulatoren oder als Pharmazeutika, insbesondere als Antimykotika und Anticonvulsiva.

In der Patentschrift GB 2 143 523 A werden 1-Aryl-1-azolylmethyl-1,3-dihydroisobenzofurane als Fungizide im Pflanzenschutz beschrieben. Jedoch sind darin weder antimykotische oder anticonvulsive Wirkungen beschrieben, noch die erfindungsgemäßen Verbindungen.

Es wurde nun gefunden, daß die erfindungsgemäßen Verbindungen, die sich von den oben erwähnten Verbindungen wesentlich durch die Art des Ringsystems unterscheiden, sehr gute antimikrobielle, insbesondere fungizide und antimykotische Eigenschaften aufweisen. Sie sind deshalb zur Bekämpfung von Pilzen bei Mensch, Tier oder Pflanzen geeignet. Durch anticonvulsive Wirkung eignen sich die erfindungsgemäßen Verbindungen des weiteren zur Behandlung und Verhütung von epileptischen Erkrankungen. Sie besitzen auch wachstumsregulierende Eigenschaften für Pflanzen.

Die Erfindung betrifft daher Azolderivate der Formel (I)

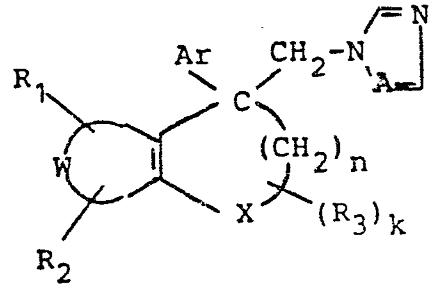

(I)

worin

A CH oder N bedeutet

k 1 oder 2 bedeutet

n 1, 2 oder 3 bedeutet

Ar Phenyl, Biphenyl, Phenoxyphenyl, Phenoxythienyl, Phenylthiophenyl, Phenylsulfinylphenyl, Phenylsulfonylphenyl, Benzylphenyl, Benzyloxyphenyl, Benzylthiophenyl, Fluorenyl, Indanyl, 1,2,3,4-Tetrahydronaphthyl, Naphthyl oder Thienyl bedeutet, wobei die Benzolringe, das Naphthalinsystem oder der Thiophenring unsubstituiert oder mit einem oder zwei Subsituenten substituiert sein können, die gleich oder verschieden sind und jeweils Fluor, Chlor, Brom, Jod, $(C_1-C_8)$-Alkyl, verzweigt oder unverzweigt, unsubstituiert oder durch 1-9 Fluor- und/oder Chloratom substituiert, $(C_3-C_8)$-Cycloalkyl, $(C_1-C_4)$-Alkoxy, $(C_1-C_4)$-Alkylthio, Hydroxy, Nitro oder Cyano bedeuten,

W die zur Vervollständigung eines Benzol-, Naphthalin- oder Thiophen-Ringsystems nötigen Atome bedeutet,

X ein Rest $>C(R_3)_2$, sowie für den Fall, daß W die zur Vervollständigung eines Benzol- oder Naphthalin-Ringsystems nötigen Atome bedeutet, auch Sauerstoff oder Schwefel bedeutet,

$R^1$ und $R^2$ gleich oder verschieden sind und jeweils Wasserstoff, Fluor, Chlor, Brom, Jod, $(C_1-C_8)$-Alkyl, unverzweigt oder verzweigt, unsubstituiert oder mit 1-9 Fluor- oder Chloratomen substituiert, $(C_3-C_8)$Cycloalkyl, $(C_1-C_8)$-Alkoxy, $(C_1-C_8)$-Alkylthio, Cyano oder Nitro bedeuten,

sowie für den Fall, daß gleichzeitig W die zur Vervollständigung eines Benzolrings nötigen Atome bedeutet,

$R^1$ auch Phenyl, Phenoxy, Phenylthio, Phenylsulfinyl, Phenylsulfonyl, Benzyl, Benzyloxy oder Hydroxy bedeutet, wobei die Phenylgruppen in diesen Substituenten unsubstituiert oder durch einen oder zwei Substituenten substituiert sein können und diese Substituenten gleich oder verschieden sind und jeweils Fluor, Chlor, Brom, Jod, $(C_1-C_4)$-Alkyl, verzweigt oder unverzweigt, unsubstituiert

oder durch 1-9 Fluor- oder Chloratome substituiert, $(C_3-C_8)$-Cycloalkyl, $(C_1-C_4)$-Alkoxy, $(C_1-C_4)$-Alkylthio, Cyano oder Nitro bedeuten, oder $R^1$, $R^2$, sofern sie orthoständig orientiert sind, zusammen eine $(C_3-C_5)$-Alkylenkette bedeuten und $R^3$ als Substituent der Kette $-(CH_2)_n$ oder von X unabhängig voneinander H oder $(C_1-C_8)$-Alkyl bedeutet oder zwei Reste $R^3$, wenn sie an dasselbe C-Atom gebunden sind, eine $(C_2-C_6)$-Alkylenkette bedeuten sowie ihre Stereoisomeren und ihre Salze.

Die als Substituenten oder in Zusammenhang mit anderen Substituenten auftretenden $(C_1-C_8)$- bzw. $(C_1-C_4)$-Alkylgruppen können unverzweigt oder verzweigt sein und beispielsweise die Methyl-, Ethyl-, Propyl-, 1-Methyl-ethyl-, Butyl-, 2-Methylpropyl-, 1,1-Dimethyl-ethyl-, Pentyl-, Hexyl-, Heptyl- oder Octylgruppe bedeuten; die durch Fluor oder Chlor substituierten Alkylgruppen können beispielsweise die Trifluormethyl-, Trichlormethyl-, 1,1,2,2-Tetrafluorethyl- oder die Nonafluorbutylgruppe bedeuten; die $(C_3-C_8)$-Cycloalkylgruppen können die Cyclopropyl-, Cyclobutyl, Cyclopentyl-, Cyclohexyl-, Cycloheptyl- oder Cyclooctylgruppe bedeuten.

Die vorliegende Erfindung betrifft die Verbindungen (I) in Form der freien Base oder eines Säureadditionssalzes mit pharmazeutisch oder für die Landwirtschaft akzeptablen Säuren.

Beispiele solcher Säuren sind anorganische Säuren wie Salzsäure, Bromwasserstoffsäure, Jodwasserstoffsäure, Schwefelsäure, Phosphorsäure, oder Salpetersäure oder organische Säuren wie Malonsäure, Oxalsäure, Gluconsäure, Camphersulfonsäure, Benzolsulfonsäure, Essigsäure, Propionsäure oder p-Toluolsulfonsäure.

Die Verbindungen (I) weisen mindestens ein asymmetrisches C-Atom auf und können daher als Enantiomere und Diastereomere auftreten. Die Erfindung umfaßt sowohl die reinen Isomeren als auch deren Gemische. Die Gemische von Diastereomeren können nach gebräuchlichen Methoden, zum Beispiel durch selektive Kristallisation aus geeigneten Lösungsmitteln oder durch

Chromatographie an Kieselgel oder Aluminiumoxid in die Komponenten aufgetrennt werden. Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Bevorzugt sind Verbindungen der Formel I, worin

W die zur Vervollständigung eines Benzol-Ringsystems nötigen Atome bedeutet.

X die Methylengruppe, Sauerstoff oder Schwefel bedeutet,

A CH oder N bedeutet,

n 1, 2 oder 3 bedeutet,

Ar Phenyl, 4-Biphenylyl, 4-Phenoxyphenyl, 4-Phenylthiophenyl, Fluorenyl, Indanyl, 1,2,3,4-Tetrahydronaphthyl oder Thienyl bedeutet, unsubstituiert oder an den Benzolringen oder am Thiophenring mit einem oder zwei Substituenten substituiert, die gleich oder verschieden sein können und jeweils Fluor, Chlor, Brom, $(C_1-C_4)$-Alkyl, $(C_3-C_8)$-Cycloalkyl, $(C_1-C_4)$-Alkoxy oder Trifluormethyl bedeuten,

$R^1$ und $R^2$ gleich oder verschieden sind und jeweils Wasserstoff, Fluor, Chlor, Brom, Trifluormethyl, $(C_1-C_8)$-Alkyl oder $(C_1-C_8)$Alkoxy bedeuten.

Von diesen Verbindungen sind von besonderer Bedeutung solche, bei denen

Ar Mono-Halogenphenyl oder 2,4-Dihalogenphenyl bedeutet, wobei die Halogenatome gleich oder verschieden sind und jeweils Fluor oder Chlor bedeuten oder Ar 4-Biphenylyl, 4-Phenoxyphenyl, 4-Phenylthiophenyl oder Fluorenyl bedeutet, unsubstituiert oder an einem Benzolring mit einem oder zwei Substituenten substituiert und diese Substituenten gleich oder verschieden sind und jeweils Fluor, Chlor, Trifluormethyl, Methyl oder Methoxy bedeuten.

Von letzteren haben insbesondere solche Interesse, worin Ar 4-Chlorphenyl, 4-Fluorphenyl, 2,4-Dichlorphenyl, 2,4-Difluorphenyl, 4-Chlor-2-fluorphenyl, Fluorenyl, 4-Biphenylyl, 4-Phenoxyphenyl oder 4-Phenylthiophenyl bedeutet, wobei die drei letztgenannten Gruppen unsubstituiert oder in der 4'-Position mit Fluor, Chlor, Methyl, Methoxy oder der Trifluormethylgruppe substituiert sein können, $R^1$ und $R^2$ gleich oder verschieden sind und Wasserstoff, Fluor, Chlor, Trifluormethyl, Methyl oder Methoxy bedeuten.

Die Erfindung betrifft weiterhin ein Verfahren zur Herstellung von Verbindungen der Formel (I), dadurch gekennzeichnt, daß man

a) eine Verbindung der Formel (II)

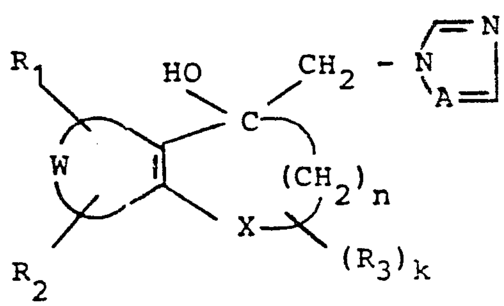

worin A, W, X, k, n, $R^1$, $R^2$ und $R^3$ die in Anspruch 1 angegebenen Bedeutungen haben, mit einer Verbindung der Formel (III)

$$Ar\text{-}H \qquad (III)$$

worin Ar die in Anspruch 1 angegebenen Bedeutungen hat, in Gegenwart einer Lewis-Säure oder einer starken Protonensäure unter Wasserabspaltung zu einer Verbindung der Formel (I) umsetzt.

Man arbeitet dabei im Temperaturbereich von -20°C bis 100°C vorzugsweise von -10° bis 50°C, gegebenenfalls in Gegenwart eines inerten Verdünnungsmittels. Unter einer Lewis-Säure oder einer starken Protonensäure sind die üblichen Friedel-Crafts-Katalysatoren zu verstehen, wie sie - ebenso wie geeignete Verdünnungsmittel - z.B. in "Houben-Weyl, Methoden

der organischen Chemie", Bd. 7/2a, S. 17-21, beschrieben sind. Bevorzugte Verdünnungsmittel sind Dichlormethan, 1,2-Dichlorethan, 1,1,2,2-Tetrachloräthan oder auch Chlorbenzol oder Dichlorbenzol; als Katalysator kommt bevorzugt Aluminiumtrichlorid zum Einsatz. Vom Katalysator setzt man 1,1 bis 2,5, bevorzugt 2,0 bis 2,3 Äquivalente bezogen auf die Verbindung der Formel (II) ein, vom Arylkohlenwasserstoff der Formel (III) zumindest 1 Äquivalent, bezogen auf die Verbindung der Formel (II), ein. Bei Verwendung der Verbindung der Formel (III) im Überschuß kann diese gleichzeitig als Verdünnungsmittel dienen.

Die Erfindung betrifft weiterhin ein Verfahren zur Herstellung von Verbindungen der Formel (I), dadurch gekennzeichnet, daß man
b) eine Verbindung der Formel (IV),

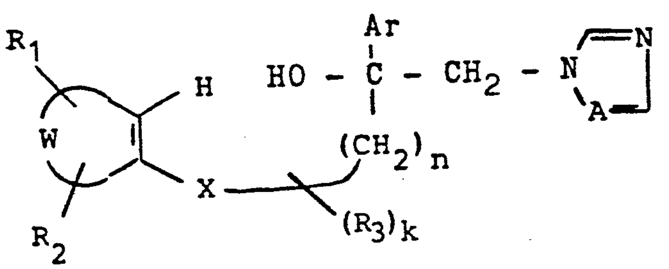

(IV)

worin A, W, X, Ar, k, n, $R^1$, $R^2$ und $R^3$ die in Anspruch 1 angegebenen Bedeutungen haben, in Gegenwart einer Lewis-Säure oder einer starken Protonen-Säure unter Wasserabspaltung zu einer Verbindung der Formel (I) umsetzt. ($R_3$ ist Substituent der $(CH_2)_n$-Kette).
Unter einer Lewis-Säure oder einer starken Protonen-Säure sind die üblichen Friedel-Crafts-Katalysatoren zu verstehen, wie sie z.B. in "Houben-Weyl, Methoden der organischen Chemie" Bd. 7/2a, S. 17-21 beschrieben sind. Bevorzugt sind dabei Aluminiumtrichlorid, 85 %ige Schwefelsäure, Polyphosphorsäure, 48 %ige Bromwasserstoffsäure oder Fluorwasserstoff.

Im einzelnen geht man dabei so vor, daß man bei Verwendung von Aluminiumchlorid als Friedel-Crafts-Katalysator die

Verbindung der Formel (IV) in einem geeigneten Lösungsmittel, vorzugsweise Dichlormethan, 1,2-Dichlorethan oder 1,1,2,2-Tetrachlorethan, in einem Temperaturbereich von -20° - 80°C, bevorzugt -10° - 50°C mit 1,1 bis 2,5, bevorzugt 2,0 bis 2,3 Äquivalenten Aluminiumchlorid zur Reaktion bringt.

Bei Verwendung von Schwefelsäure, Polyphosphorsäure, Bromwasserstoffsäure oder Fluorwasserstoffsäure werden die jeweiligen Säuren im großen Überschuß eingesetzt und dienen gleichzeitig als Reaktionsmedium. Im Falle von Schwefelsäure arbeitet man im Temperaturbereich von -10° - 50°C, vorzugsweise von 0° - 30°C, bei Verwendung von Polyphosphorsäure oder Bromwasserstoffsäure im Bereich von 60 - 150°C, vorzugsweise von 80 - 120°C, im Falle von Fluorwasserstoff im Bereich von -10° - 100°C, vorzugsweise zwischen 30° - 60°C, nötigenfalls unter Verwendung eines Autoklaven.

Die Erfindung betrifft weiterhin ein Verfahren zur Herstellung von Verbindungen der Formel (I), dadurch gekennzeichnet, daß man

c) eine der nach a) oder b) erhaltenen Verbindungen der Formel (I), für die Ar eine mit einer Alkoxygruppe substituierte Phenyl- oder Naphthylgruppe bedeutet oder für die $R^1$ eine Alkoxygruppe bedeutet, einer Etherspaltung unterwirft und die so erhaltenen Verbindungen der Formel (I), für die $R^1$ die Hydroxygruppe bedeutet oder für die Ar eine mit einer Hydroxygruppe substituierte Phenyl- oder Naphthylgruppe bedeutet, mit einer Verbindung der Formel (V),

$$E - R^4 \qquad (V)$$

worin E eine reaktive Abgangsgruppe bedeutet und $R^4$ eine $(C_1-C_8)$-Alkylgruppe oder Benzylgruppe nach Anspruch 1 bedeutet, umsetzt. Die Etherspaltung wird in üblicher Weise

durch Erhitzen der Alkoxyverbindung mit Bromwasserstoffsäure durchgeführt. Die Veretherung mit der Verbindung (V), worin die reaktive Abgangsgruppe E beispielsweise Chlor, Brom, Jod oder eine Sulfonyloxygruppe wie 4-Toluol-, 4-Chlorphenyl- oder Methylsulfonyloxy bedeutet, erfolgt in üblicherWeise unter den Bedingungen einer Williamsonschen Ethersynthese (vgl. "Organikum, Organisch-Chemisches Grundpraktikum" Berlin 1969, S. 223-226) oder auch unter den Bedingungen einer Phasentransferreaktion, indem man die Reaktionspartner in einem aromatischen Kohlenwasserstoff wie Benzol, Chlorbenzol, Toluol oder Xylol unter kräftigem Rühren in Gegenwart eines Phasentransferkatalysators und entweder einem gepulverten Alkalihydroxid, wie z.B. Natrium- oder Kaliumhydroxid oder einer konzentrierten wäßrigen Lösung derselben, vorzugsweise in einem Temperaturbereich von 20 bis 120°C, aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4-, 15-Crown-5, 18-Crown-6 oder Dibenzo-18-crown-6.

Die Erfindung betrifft weiterhin ein Verfahren zur Herstellung von Verbindungen der Formel (I), dadurch gekennzeichnet, daß man

d) eine Verbindung der Formel (I) an einer Phenylthiogruppe- oder Phenylsulfonylgruppe oxidiert.

Geeignete Oxidationsmittel für die Oxidation zum Sulfoxid sind z.B. Wasserstoffperoxid oder Persäuren wie Peressigsäure oder m-Chlorperbenzoesäure oder Natrium- oder Kaliummetaperjodat; für die Oxidation zum Sulfon sind mit Ausnahme der Metaperjodate die gleichen Oxidationsmittel geeignet. Geeignete Lösungsmittel für die Oxidation mit Persäuren

sind z.B. Methylenchlorid oder Chloroform, für die Oxidation mit Perjodaten Alkohol/Wasser-Gemische. Der bevorzugte Temperaturbereich liegt zwischen -20° und 50°C.

Gegenstand der Erfindung sind weiterhin Verbindungen der Formel (II), sowie ihre Salze und ihre Stereoisomeren.

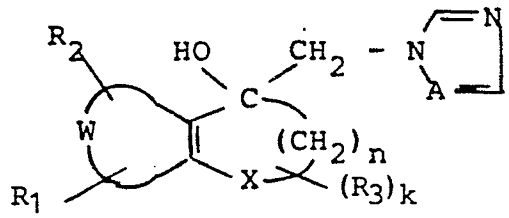

(II)

worin A, W, X, n, K, $R^1$, $R^2$ und $R^3$ die in Anspruch 1 angegebenen Bedeutungen haben, mit der Einschränkung, daß (II) nicht die Verbindungen 1-Hydroxy-1-(azol-1-ylmethyl)-1,2,3,4-tetrahydro-naphthalin bedeutet, wobei unter "Azol" 1H-Imidazol oder 1,2,4-Triazol zu verstehen ist.

Die Verbindungen der Formel (II) zeigen antimikrobielle, insbesondere antimykotische Eigenschaften. Sie sind deshalb zur Bekämpfung von Pilzen bei Mensch und Tier geeignet. Durch anticonvulsive Wirkung eignen sich die Verbindungen der Formel (II) des weiteren zur Behandlung und Verhütung von epileptischen Erkrankungen. Durch fungizide und wachstumsregulierende Eigenschaften sind sie auch als Pflanzenschutzmittel geeignet.

Die vorliegende Erfindung betrifft die Verbindungen (II) in Form der freien Base oder eines Säureadditionssalzes oder eines physiologisch hydrolysierbaren und akzeptablen Derivates.

Beispiele pharmazeutisch akzeptabler salzbildender Säuren sind anorganische Säuren wie Salzsäure, Bromwasserstoffsäure, Jodwasserstoffsäure, Schwefelsäure, Phosphorsäure, oder Salpetersäure oder organische Säuren wie Malonsäure,

Oxalsäure, Gluconsäure, Camphersulfonsäure, Benzolsulfonsäure, Essigsäure, Propionsäure oder p-Toluolsulfonsäure.

Als physiologisch hydrolysierbare und akzeptable Derivate sind beispielsweise an der Hydroxygruppe veresterte Derivate geeignet, die unter physiologischen Bedingungen zu den freien Säuren hydrolysierbar sind, die ihrerseits wieder physiologisch akzeptierbar sind, d.h. bei den notwendigen Dosen nicht toxisch sind.

Die Verbindungen (II) weisen mindestens ein asymmetrisches C-Atom auf und können daher als Enantiomere und Diastereomere auftreten. Die Erfindung umfaßt sowohl die reinen Isomeren als auch deren Gemische. Die Gemische von Diastereomeren können nach gebräuchlichen Methoden, zum Beispiel durch selektive Kristallisation aus geeigneten Lösungsmitteln oder durch Chromatographie an Kieselgel oder Aluminiumoxid in die Komponenten aufgetrennt werden. Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Gegenstand der Erfindung ist weiterhin ein Verfahren zur Herstellung von Verbindungen der Formel (II), dadurch gekennzeichnet, daß man eine Verbindung der Formel (VI)

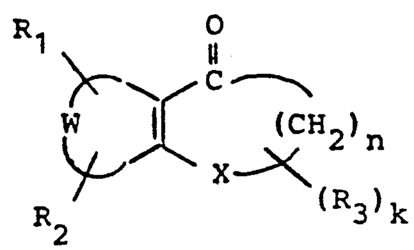

(VI)

worin W, X, k, n, $R^1$, $R^2$ und $R^3$ die in Anspruch 1 angegebenen Bedeutungen haben, mit einem Schwefelylid der Formel (VII),

$$(CH_3)_2 \underset{\underset{(O)_z}{\|}}{S} CH_2 \qquad (VII)$$

worin z 0 oder 1 bedeutet,
zu einer Verbindung der Formel (VIII) umsetzt

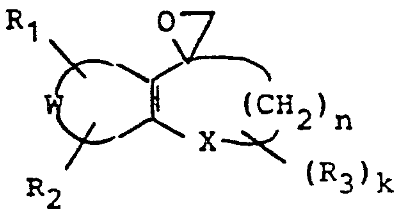

(VIII)

und anschließend die Verbindung der Formel (VIII) mit einer Verbindung der Formel (IX)

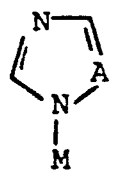

(IX)

worin A die in Anspruch 1 angegebenen Bedeutungen hat und M Wasserstoff, die Trimethylsilylgruppe oder ein Metalläquivalent bedeutet, zu einer Verbindung der Formel (II) umsetzt.

Hierzu wird in einem ersten Reaktionsschritt zur Herstellung der Verbindungen der Formel (VIII) ein Keton der Formel (VI), in der $R^1$, $R^2$, $R^3$, W, X, k und n die oben angegebenen Bedeutungen haben, mit einem Schwefelylid der Formel (VII) in einem inerten Lösungsmittel, vorzugsweise Dimethylsulfoxid, oder in Gemischen von Dimethylsulfoxid mit anderen inerten Lösungsmitteln, z.B. Tetrahydrofuran, umgesetzt. Dabei ist bei Verwendung eines Schwefelylids der Formel (VII), für das z = 0 ist, ein Temperaturbereich zwischen -10° und 50°C, vorzugsweise zwischen 0° und 30°C zweckmäßig;

bei Verwendung eines Schwefelylids der Formel (VII), für das z = 1 ist, ein Temperaturbereich zwischen 0° und 80°, vorzugsweise zwischen 20° und 50°.

Die Herstellung der Ketone der Formel (VI) oder der Schwefelylide (VII) erfolgt nach in der Literatur beschriebenen Verfahren. So werden die Verbindungen (VI) beispielsweise wie in Org.Synth.Coll.Vol. IV S. 898 oder loc.cit.Vol. II S. 569 hergestellt.

Die Umwandlung der Zwischenprodukte der Formel (VIII) in die Endprodukte der Formel (II) erfolgt in einem zweiten Reaktionsschritt durch Umsetzung mit einer Verbindung der Formel (IX), in der A und M die oben angegebenen Bedeutungen haben, in einem inerten Lösungsmittel, z.B. N,N-Dimethylformamid, N,N-Dimethylacetamid, Dimethylsulfoxid, Acetonitril, Benzonitril, Sulfolan, N-Methylpyrrolidon, Dioxan oder Tetrahydrofuran, in einem Temperaturbereich zwischen 20° und 150°C.

Die Verbindung (IX) kann dabei in Form eines Alkali- oder Erdalkalimetallsalzes, bevorzugt als Natriumsalz, oder in Form der freien Base in Gegenwart einer starken Base, umgesetzt werden. Geeignete Basen sind z.B. Alkali- oder Erdalkalimetallhydride, -amide oder alkoholate.

Der zweite Reaktionsschritt kann auch so durchgeführt werden, daß man die Verbindung der Formel (VIII) mit Imidazol oder Triazol in äquivalenten Mengen, oder auch mit Imidazol oder Triazol im Überschuß, ohne Lösungsmittel in einem Temperaturbereich von 90 - 160°C zur Reaktion bringt.

Das oben beschriebene Verfahren kann zweckmäßig auch als Eintopfverfahren durchgeführt werden, indem man eine Verbindung der Formel (VI) mit einem Schwefelylid der Formel (VII) wie oben beschrieben zu einer Verbindung der Formel (VIII) umsetzt und diese anschließend ohne Aufarbeitung des Reaktionsgemisches und ohne Isolierung der Verbindung der Formel (VIII) durch Zugabe einer Verbindung

der Formel (IX) zum Reaktionsgemisch zu einer Verbindung der Formel (II) umsetzt und diese in Form der freien Base oder eines Säureadditionssalzes oder eines physiologisch hydrolysierbaren und akzeptablen Derivats isoliert.

Gegenstand der Erfindung sind weiterhin Verbindungen der Formel (IV)

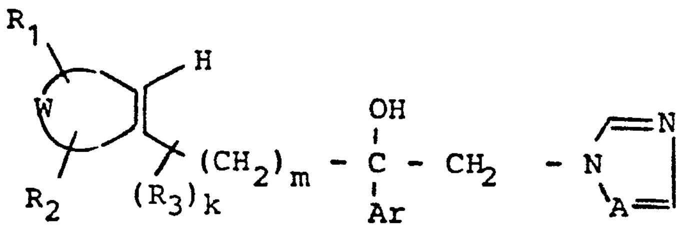

(IV)

worin A, Ar, W, k, $R^1$, $R^2$ und $R^3$ die in Anspruch 1 angegebenen Bedeutungen haben und m 3 oder 4 bedeutet. $R_3$ ist in dieser Formel ein Substituent der Kette $-(CH_2)_m-$.

Diese als Zwischenprodukte zur Herstellung der Verbindungen der Formel (I) benötigten Verbindungen zeigen ebenfalls antimikrobielle, insbesondere antimykotische Eigenschaften und sind deshalb zur Bekämpfung von Pilzen bei Mensch und Tier geeignet. Weiterhin besitzen die Verbindungen fungizide und wachstumsregulierende Eigenschaften und sind deshalb auch als Pflanzenschutzmittel geeignet.

Gegenstand der Erfindung ist weiterhin ein Verfahren zur Herstellung von Verbindungen der Formel (IV), dadurch gekennzeichnet daß man eine Verbindung der Formel (X)

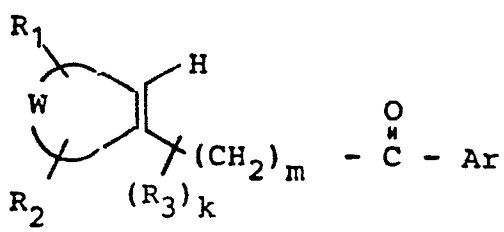

(X)

worin W, Ar, k, $R^1$, $R^2$ und $R^3$ die in Anspruch 1 angegebenen Bedeutungen haben und m 3 oder 4 bedeutet, mit einem Schwefelylid der Formel (VII) nach Anspruch 7 zu einer Verbindung der Formel (XI) umsetzt

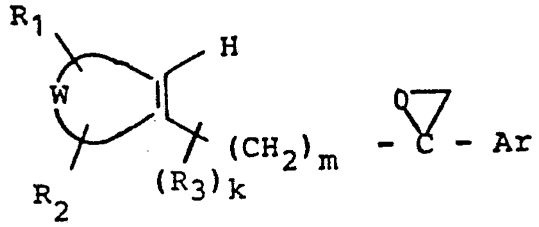

(XI)

und die Verbindung der Formel (XI) anschließend mit einer Verbindung der Formel (IX) nach Anspruch 7 zu einer Verbindung der Formel (IV) umsetzt.

Dabei verfährt man so, daß man, wie oben beschrieben, eine Lösung des Schwefelylids der Formel (VII) in einem der oben beschriebenen Lösungsmittel herstellt und dieses, wie oben bei der Umsetzung einer Verbindung der Formel (VII) mit einer Verbindung der Formel (VI) beschrieben, mit einem Keton der Formel (X) zu einem Epoxid der Formel (XI) umsetzt und dieses dann, wie oben bei der Umsetzung der Verbindung der Formel (VIII) zu einer Verbindung der Formel (II) beschrieben, mit einer Verbindung der Formel (IX) zu einer Verbindung der Formel (IV) umsetzt. Die Überführung einer Verbindung der Formel (X) in eine Verbindung der Formel (IV) kann zweckmäßig (analog der Überführung der Verbindung der Formel (VI) in eine Verbindung der Formel (II)), ohne Isolierung der Zwischenstufe der Formel (XI), als Eintopfverfahren durchgeführt werden.

Die Erfindung betrifft weiterhin ein Verfahren zur Herstellung von Verbindungen der Formel (X)

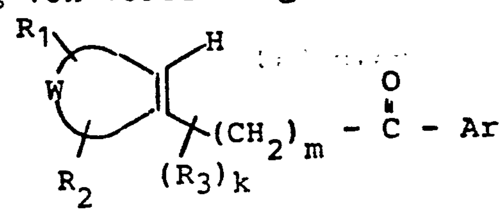

(X)

worin W, Ar, k, $R^1$ und $R^2$ die in Anspruch 1 angegebenen Bedeutungen haben und m= 3 ist, dadurch gekennzeichnet, daß man eine Verbindung der Formel (XII)

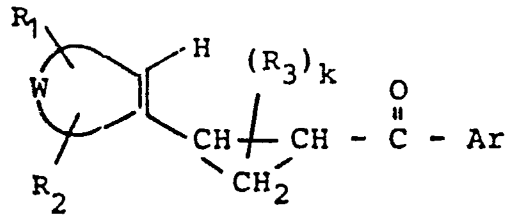

(XII)

in Gegenwart eines Metallkatalysators hydriert und so eine Verbindung der Formel (X) erhält.

Dabei verfährt man so, daß man eine Verbindung der Formel (XII) in einem inerten Lösungsmittel unter Zusatz eines Hydrierkatalysators in einem Druckbereich von 1-10 bar, bevorzugt aber bei 1 bar, in einem Temperaturbereich zwischen 0 und 100°C, bevorzugt 20-50°C, mit Wasserstoff umsetzt und dabei unter Ringöffnung des Cyclopropanrings eine Verbindung der Formel (X) erhält. Als Lösungsmittel kommen alle gegen Hydrierung beständigen und den Katalysator nicht desaktivierenden Lösungsmittel in Frage, z.B. Methanol, Ethanol, Tetrahydrofuran, Hexan, Toluol oder auch Gemische dieser Lösungsmittel. Als Metallkatalysatoren kommen die üblichen Hydrierkatalysatoren in Frage, wie sie z.B. in "Houben-Weyl, Methoden der Organischen Chemie", Bd. 4/1c, S. 18/19 beschrieben sind, bevorzugt aber Palladium auf Aktivkohle.

Die Ausgangsprodukte für die vorstehend beschriebenen Verfahren sind bekannt oder können analog beschriebener Verfahren hergestellt werden.

Für Verbindungen der Formel (X), soweit sie nicht nach Anspruch 10 hergestellt werden, sind im folgenden einige Synthesemöglichkeiten für den Fall angegeben, daß W die zur Vervollständigung eines Benzol-Ringsystems benötigten Atome bedeutet:

a)

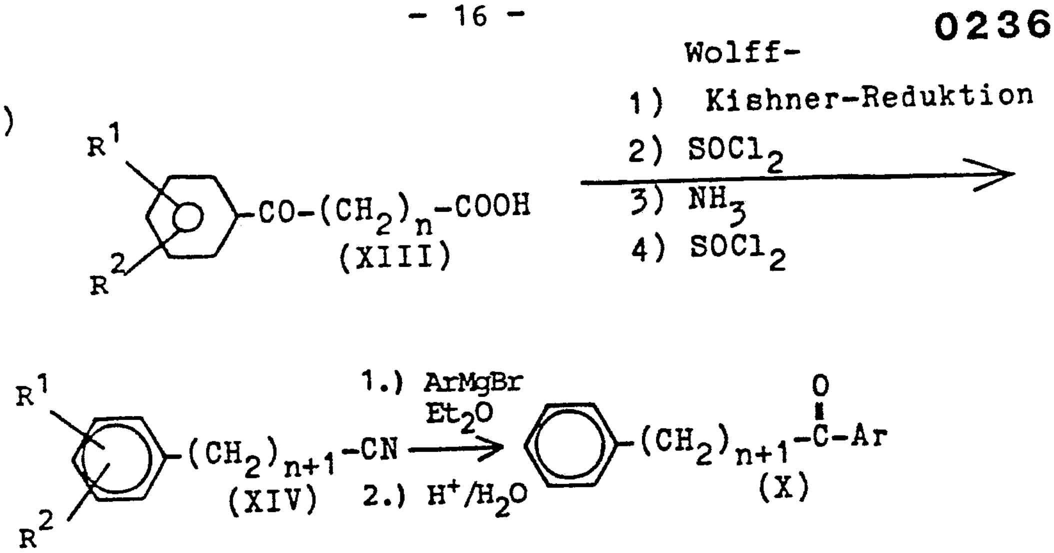


(XIII)

(XIV)

(X)

b)

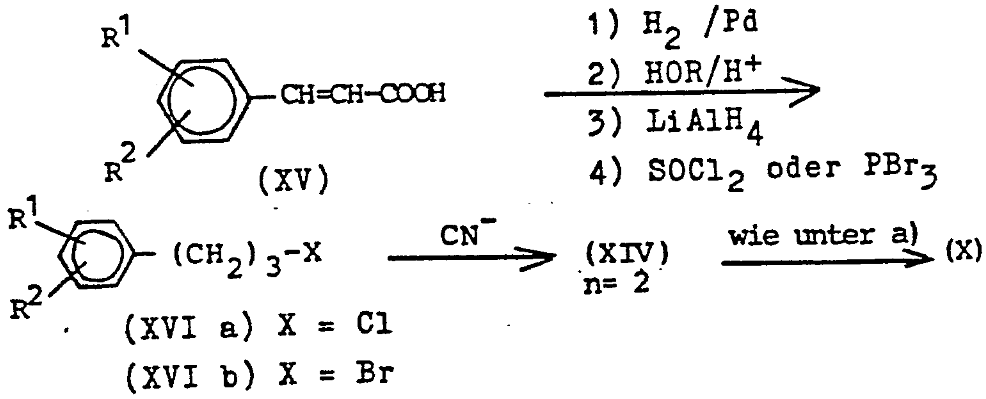


(XV)

(XIV) n= 2

(X)

(XVI a) X = Cl

(XVI b) X = Br

c)

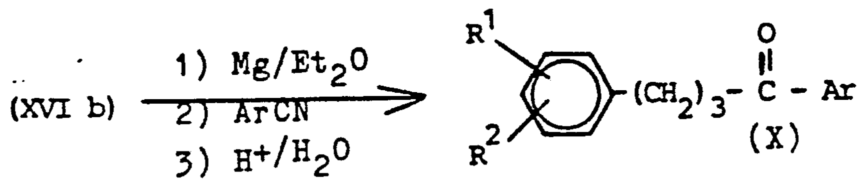


(XVI b)

(X)

Außer nach dem in Anspruch 9 beschriebenen Verfahren zur Herstellung der Verbindungen der Formel (IV) können die letzteren noch nach folgenden Methoden hergestellt werden (Beispiele für den Fall, daß W die zur Vervollständigung eines Benzol-Ringsystems benötigten Atome bedeutet):

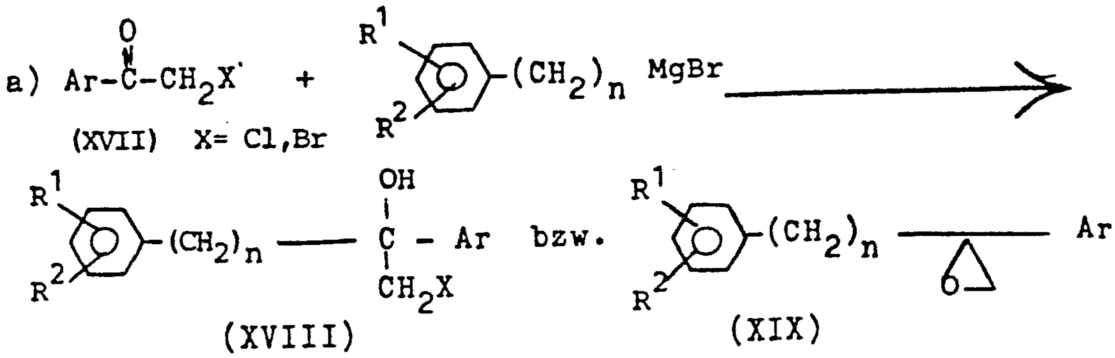

(XVII) X= Cl,Br

(XVIII) (XIX)

(XVIII), (XIX) (IX) (IV)

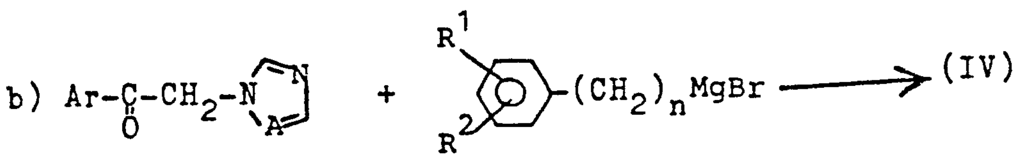

(XX)

Die Verbindungen der Formel I und ihre Säureadditionssalze sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter

Effekt gegen verschiedene Erreger der Hautmykosen (z.B. Trichophyton mentagrophytes) am Meerschweinchen nach oraler, parenteraler oder insbesondere lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Die Verbindungen der Formel (I) und (II) zeigen neben ihren antimikrobiellen Eigenschaften auch ZNS-Wirkung, insbesondere antikonvulsive Wirkung. Sie sind in den für Antiepileptika spezifischen Testanordnungen gut wirksam. So zeigen sie starke Wirkung gegen die durch Pentetrazol- oder Elektroschock ausgelösten Krämpfe bei Mäusen.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen vorzugsweise in einer Konzentration von etwa 0,01 bis 99,0, vorzugsweise von etwa 0,05 bis 50 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.
Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von mindestens etwa 0,05, vorzugsweise 0,1, insbesondere 0,5 mg und von höchstens 200, vorzugsweise 100, insbesondere

0236913

30 mg/kg Körpergewicht je 24 Stunden, bezogen auf einen einen Erwachsenen des Gewichts von 75 kg, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen der Formel (I) zeichnen sich weiterhin durch eine hervorragende fungizide Wirkung bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Pellicularia sasakii, verschiedene Rostarten, vor allem aber Venturia inaequalis, Cercospora-Arten und echte Mehltaupilze im Obst-, Gemüse-, Getreide- und Zierpflanzenbau.

Die neuen Verbindungen der allgemeinen Formel (I) eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel z.B. ligninsulfonsaures Natrium, 2,2'-dinapthylmethan-6,6'-di-sulfonsaures Natrium, dibutylnaphthalin-sulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze, wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fett-

alkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-% besteht aus üblichen Formulierungsbestandteilen. Bei emulgierbaren Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die benötigten Aufwandmengen können je nach Indikation innerhalb weiter Grenzen schwanken und variieren auch in Abhängigkeit von äußeren Faktoren wie Bodenverhältnissen, Klimabedingungen. Im allgemeinen liegen die Dosismengen zwischen 0,01 und 10 kg Wirkstoff pro ha, insbesondere zwischen 0,15 und 1,0 kg pro ha.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispegierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

Die nachfolgenden Beispiele dienen zur näheren Erläuterung der Erfindung, ohne dieselbe einzuschränken.

Experimenteller Teil

Beispiel 1

6,7-Dichlor-4-(4-chlorphenyl)-4-(1H-imidazol-1-ylmethyl)-2,3-dihydro-benzopyran

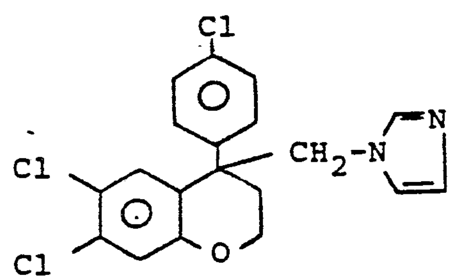

Eine Lösung von 4,6 g (0,011 Mol) 2-(4-Chlorphenyl)-4-(3,4-dichlorphenoxy)-1-(1H-imidazol-1-yl)-butan-2-ol (Beispiel 17) in 150 ml 1,2-Dichlorethan wurde bei -10°C unter Rühren mit 3,1 g (0,023 Mol) wasserfreiem Aluminiumchlorid versetzt. Man ließ auf Raumtemperatur kommen und rührte weitere 6 h. Anschließend wurde auf Eis gegossen, mit 2n Natronlauge basisch gestellt, die organische Phase mit Wasser gewaschen, getrocknet und eingeengt. Das Rohprodukt wurde an Kieselgel mit Ethylacetat/Methanol 9:1 chromatographiert. Man erhielt ein farbloses Öl, das beim Verreiben mit Diethylether kristallisierte.

Ausbeute: 2,8 g (60,8 % d.Th.)

Fp.: 94°C Zers. (Produkt kristallisiert mit 1/3 Diethylether)

Beispiel 2

7-Chlor-4-(4-chlorphenyl)-4-(1,2,4-triazol-1-ylmethyl)-2,3-dihydro-pyran

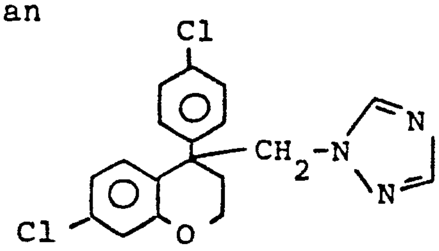

Analog Beispiel 1 wurden 4,0 g (0,011 Mol) 2-(4-Chlorphenyl)-4-(3-chlorphenoxy)-1-(1,2,4-triazol-1-yl)-butan-2-ol (Beispiel 18) mit 3,0 g (0,22 mol) Aluminiumchlorid in 150 ml 1,2-Dichlorethan umgesetzt. Zur Reinigung wurde an Kieselgel mit Methylenchlorid/Ethylacetat 2:1 chromatographiert. Man erhielt 2,5 g glasartiges Produkt (66,1 % d.Th.).

Beispiel 3

7-Chlor-4-(4-chlorphenyl)-4-(1H-imidazol-1-ylmethyl)-2,3-dihydro-thiopyran

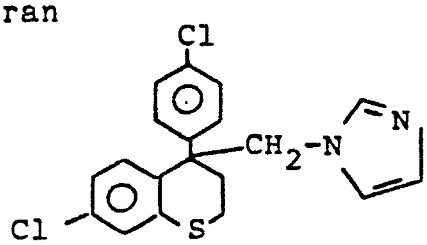

Analog Beispiel 1 wurden 3,9 g (0,010 Mol) 2-(4-Chlorphenyl)-1-(1H-imidazol-1-yl)-4-(3-chlorphenylthio)-butan-2-ol (Beispiel 21) mit 2,7 g (0,020 Mol) Aluminiumchlorid in 100 ml 1,2-Dichlorethan umgesetzt. Nach Chromatographie an Kieselgel mit Ethylacetat/Methanol 19:1 erhielt man ein farbloses Harz (2,3 g, 61,3 % d.Th.) das beim Verreiben mit Diethylether kristallisierte.

Fp.: 143 - 144°C.

0236913

Beispiel 4

1-(1H-Imidazol-1-ylmethyl)-1-(4-trifluormethylphenyl)-tetralin

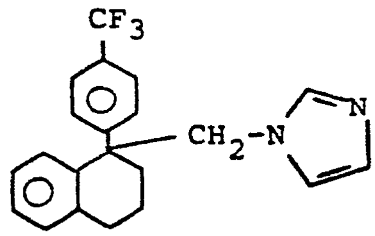

Bei Raumtemperatur wurden 7,0 g (0,019 Mol) 1-(1H-Imidazol-1-yl)-5-phenyl-2-(4-trifluormethylphenyl)-pentan-2-ol (Beispiel 22) in 30 ml 85 %ige Schwefelsäure eingetragen. Man rührte 2h bei Raumtemperatur, goß auf Eis, stellte mit 10 %iger Natronlauge basisch und schüttelte mit Methylenchlorid aus. Die organische Phase wurde getrocknet, eingeengt und der Rückstand an Kieselgel chromatographiert (Ethylacetat/Methanol 9:1). Man erhielt 4,7 g (70,5 % d.Th.) eines farblosen Harzes.

Beispiel 5

1-(4-Bromphenyl)-1-(1,2,4-triazol-1-ylmethyl )-tetralin

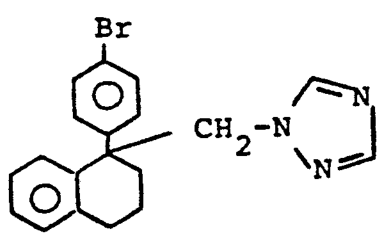

Zu einer Suspension von 11,6 g (0,03 Mol) 2-(4-Bromphenyl)-5-phenyl-1-(1,2,4-triazol-1-yl)-pentan-2-ol (Beispiel 24) in 200 ml 1,2-Dichlorethan gab man bei Raumtemperatur 8,4 g (0,063 Mol) Aluminiumchlorid. Nach Abklingen der exothermen Reaktion wurde 4 h bei Raumtemperatur nachgerührt, auf Eis gegossen, mit Natronlauge basisch gestellt, die organische Phase abgetrennt, getrocknet und eingeengt. Das Rohprodukt wurde an Kieselgel mit Ethylacetat chromatographiert. Man erhielt 7,2 g (65,2 %) farbloses Öl, das beim Verreiben mit Diethylether kristallisierte.
Fp.: 121°C

Beispiel 6

1-[4-(4-Chlorbenzyloxy)-phenyl]-1-(1H-imidazol-1-ylmethyl)-tetralin

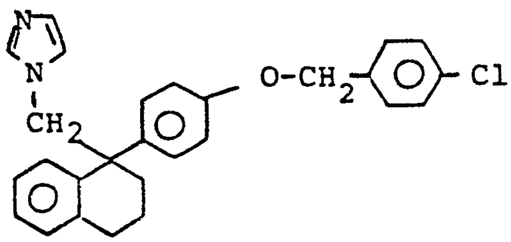

Ein Gemisch aus 1,5 g (4,9 mMol) 1-(4-Hydroxyphenyl)-1-(1H-imidazol-1-ylmethyl (Beispiel 7), 0,3 g Tetrabutylammoniumbromid, 0,8 g (4,9 mMol) 4-Chlorbenzylchlorid, 5 ml 50 %iger Natronlauge und 30 ml Toluol wurde 5 h unter heftigem Rühren unter Rückfluß erhitzt. Zur Aufarbeitung wurde mit Wasser verdünnt, die organische Phase abgetrennt, die Wasserphase nochmals mit Methylenchlorid ausgeschüttelt und die vereinigten organischen Phasen getrocknet und eingeengt. Nach Chromatographie an Kieselgel (Ethylacetat/Methanol 9:1) wurde ein Öl erhalten, das beim Verreiben mit Ethylacetat kristallisierte.
Ausbeute: 1,4 g (66 % d.Th.)
Fp: 132 - 133°C

Beispiel 7

1-(4-Hydroxyphenyl)-1-(1H-imidazol-1-ylmethyl)-tetralin

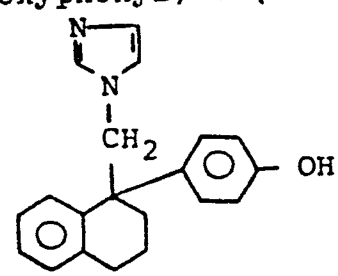

5,0 g (0,015 Mol) 1-(1H-imidazol-1-yl)-5-phenyl-2-(4-methoxyphenyl)-pentan-2-ol (Verbindung Nr. 20, Tabelle 2)

wurde mit 50 ml 48 %iger Bromwasserstoffsäure 6 h unter Rückfluß erhitzt. Zur Aufarbeitung wurde die Säure im Vakuum abgezogen, der feste Rückstand mit wäßriger Ammoniaklösung erhitzt und abgesaugt. Man erhielt 4,2 g (93,1 % d.Th.) farblosen Feststoff.
Fp: 272 - 275°C

Beispiel 8

5-(4-Chlorbenzyloxy)-1-(2,4-dichlorphenyl)-1-(1H-imidazol-1-ylmethyl)-tetralin

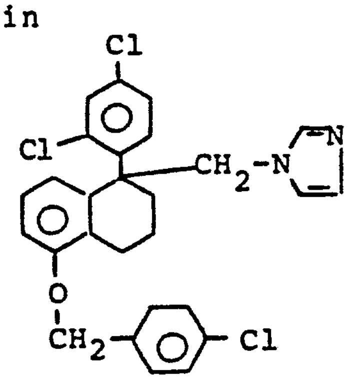

Analog Beispiel 6 wurden 1,40 g (3,9 mMol 1-(2,4-Dichlorphenyl)-5-hydroxy-1-(1H-imidazol-1-ylmethyl)-tetralin (Beispiel 9), mit 0,63 g (3,9 mMol) Chlorbenzylchlorid unter Zusatz von 0,30 g Tetrabutylammoniumbromid in einem Gemisch aus 50 ml Toluol und 5 ml 50 %iger Natronlauge umgesetzt. Nach Chromatographie an Kieselgel erhielt man 0,8 g farblose Kristalle (41,2 % d.Th.).
Fp: 184 - 185°C

Beispiel 9

1-(2,4-Dichlorphenyl)-5-hydroxy-1-(1H-imidazol-1-ylmethyl)-tetralin

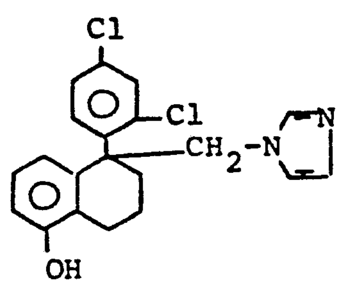

1,7 g (4,4 mMol) 1-(2,4-Dichlorphenyl)-1-(1' 'ida"ol-1-

ylmethyl)-5-methoxy-tetralin (Verbindung Nr. 42, Tabelle 1, hergestellt analog Beispiel 5 aus 2-(2,4-Dichlorphenyl)-1-(1H-imidazol-1-yl)-5-(2-methoxyphenyl)-pentan-2-ol, Verbindung Nr. 26, Tabelle 2) wurden in 30 ml Eisessig/ 48 %ige Bromwasserstoffsäure (1:1) 6h unter Rückfluß erhitzt. Nach Abkühlen wurde das Säuregemisch im Vakuum abgezogen, der feste Rückstand kurz mit wäßriger Ammoniaklösung erhitzt und abgesaugt. Man erhielt 1,4 g (89,2 % d.Th.) farblosen Feststoff.
Fp: 245°C Zers.

Beispiel 10

1-(4-Chlorphenyl)-1-(1H-imidazol-1-ylmethyl)-indan

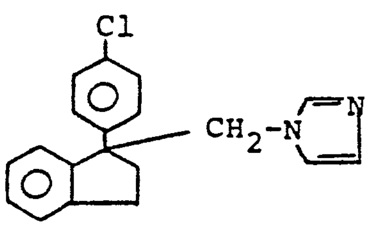

In einem 10 ml V4A-Autoklaven wurden 2,5 g (7,6 mMol) 2-(4-Chlorphenyl)-1-(1H-imidazol-1-yl)-4-phenyl-butan-2-ol (Beispiel 26) in 2,5 ml wasserfreier Flußsäure unter Argonatmosphäre 24 h bei 50°C gerührt. Bei 0°C wurde auf 100 g Eis gegossen, mit 40 %iger Kalilauge auf pH 10 eingestellt und viermal mit je 50 ml Methylenchlorid extrahiert. Die vereinigten organischen Phasen wurden getrocknet und eingeengt.

Chromatographie an Kieselgel mit Ethylacetat/Methanol 9:1 ergab 1,0 g eines farblosen Harzes (42,6 % d.Th.)

Beispiel 11

1-(3,4-Dimethylphenyl)-1-(1H-imidazol-1-ylmethyl)-tetralin

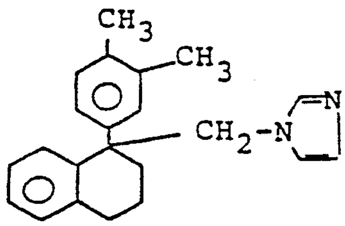

5,7 g fein pulverisiertes 1-Hydroxy-1-(1H-imidazol-1-yl-methyl)-tetralin (0,025 Mol) (Beispiel 33) in einem Gemisch aus 100 ml o-Xylol/Methylenchlorid 1:1 wurde bei Raumtemperatur mit 7,0 g (0,53 Mol) Aluminiumchlorid versetzt. Nach Abklingen der exothermen Reaktion wurde eine Stunde bei Raumtemperatur nachgerührt. Zur Aufarbeitung wurde auf Eis gegossen, mit Natronlauge basisch gestellt, die organische Phase getrocknet und im Vakuum eingeengt. Nach Chromatographie an Kieselgel (Ethylacetat/Methanol 9:1) verblieb ein farbloses Öl, das beim Verreiben mit Cyclohexan kristallisierte.

Ausbeute: 3,6 g (45,5 %)

Fp: 102 - 103°C

Beispiel 12

1-(3,4-Dimethylphenyl)-1-(1H-imidazol-1-ylmethyl)-2,3,4,5-tetrahydro-benzocycloheptatrien

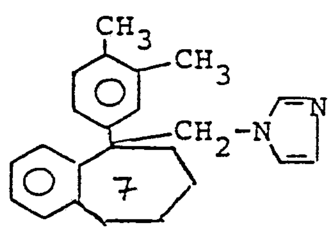

Analog Beispiel 11 wurden 9,7g (0,04 Mol) 1-Hydroxy-1-(1H-imidazol-1-ylmethyl)-2,3,4,5-tetrahydro-benzocycloheptatrien (Verbindung Nr. 3, Tabelle 3) in einem Gemisch aus 150 ml o-Xylol/1,2-Dichlorethan 1:1 durch Zugabe von 11,1 g (0,083 Mol) Aluminiumchlorid umgesetzt. Man arbeitet analog Beispiel 11 auf. Nach

0236913

Chromatographie an Kieselgel mit Ethylacetat erhielt man ein farbloses Öl, das beim Verreiben mit Diethylether kristallisierte.
Ausbeute: 1,9 g (14,4 % d.Th.)
Fp: 158 - 160°C

Beispiel 13
1-(4-Biphenylyl)-1-(1H-imidazol-1-ylmethyl)-indan

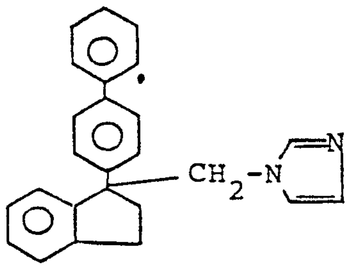

Zu einer auf 0°C gekühlten Suspension von 10,0g(0,047 Mol) 1-Hydroxy-1-(1H-imidazol-1-ylmethyl)-indan (Verbindung Nr. 1, Tabelle 3) in einem Gemisch aus 60 g Biphenyl und 60 ml 1,2-Dichlorethan gab man 13,1 g (0,98 Mol) Aluminiumchlorid. Nach Abkühlen der exothermen Reaktion wurde eine Stunde nachgerührt. Man arbeitete analog Beispiel 11 auf. Nach Chromatographie an Kieselgel mit Ethylacetat/Methanol 9:1 erhielt man ein farbloses Öl, das beim Verreiben mit Diethylether kristallisierte.
Ausbeute: 10,7 g (65,4 % d.Th.)
Fp: 123 - 125°C

Beispiel 14
1-(1H-Imidazol-1-ylmethyl)-1-(4-phenylthiophenyl)-tetralin

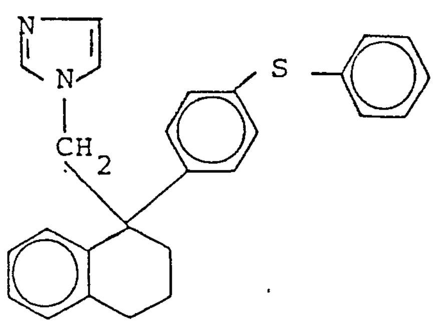

Zu einer auf 0°C gekühlten Suspension von 6,5 g (0,029 Mol) fein pulverisiertem 1-Hydroxy-1-(1H imidazol-1-ylmethyl)-tetralin (Beispiel 33) in einem Gemisch aus 25 ml Diphenylsulfid und 50 ml 1,2-Dichlorethan wurden 8,0 g (0,06 Mol) Aluminiumchlorid gegeben. Nach Abklingen der exothermen Reaktion wurde 3 h bei Raumtemperatur nachgerührt, das Gemisch auf Eis gegossen und mit Natronlauge alkalisch gestellt. Es wurde mit Methylenchlorid ausge-

schüttelt, getrocknet und die organische Phase eingeengt. Nach Chromatographie an Kieselgel (Ethylacetat/Methanol 9:1) erhielt man ein farbloses Öl, das beim Verreiben mit Diethylether kristallisierte.
Ausbeute: 8,5 g (72,2 % d.Th.)
Fp: 124°C

Beispiel 15

1-(1H-Imidazol-1-ylmethyl)-1-(4-phenylsulfinylphenyl)-tetralin

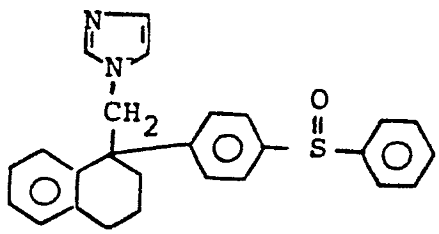

Zu einer Lösung von 3,0 g (7,6 mMol) 1-(1H-Imidazol-1-ylmethyl)-1-(4-phenylthiophenyl)-tetralin (Beispiel 14) in 80 ml Methylenchlorid tropfte man bei -10°C eine Lösung von 1,7 g (7,6 mMol) 70 %iger 3-Chlorperbenzoesäure in 80 ml Methylenchlorid. Nach Stehen über Nacht wurde mit Natriumhydrogencarbonatlösung verrührt, die organische Phase getrocknet und eingeengt. Chromatographie am Kieselgel (Ethylacetat/Methanol 9:1) ergab 2,6 g farbloses Harz (82,9 % d.Th.).

Beispiel 16

1-(1H-Imidazol-1-ylmethyl)-1-(4-phenylsulfonylphenyl)-tetralin

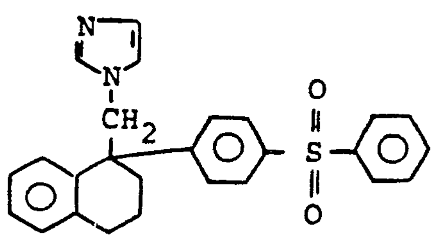

Zu einer Lösung von 3,5 g (8,8 mMol) 1-(1H-Imidazol-1-ylmethyl)-1-(4-phenylthiophenyl)-tetralin (Beispiel 14) in 100 ml Methylenchlorid tropfte man bei 0°C eine Lösung von 3,4 g (17,6 mMol) 70 %ige m-Chlorperbenzoesäure in 80 ml Methylenchlorid. Nach Stehen über Nacht wurde mit Natriumhydrogencarbonatlösung verrührt, die organische Phase getrocknet und eingeengt. Chromatographie an Kieselgel (Ethylacetat/Methanol 9:1) ergab ein farbloses Harz, das beim Verreiben mit Diethylether kristallisierte.
Ausbeute: 3,0 g (79,5 % d.Th.)
Fp: 143 - 144°C

Beispiel 16a
1-(2-Chlor-thien-5-yl)-1-(1H-imidazol-1-ylmethyl)-3,3-dimethyl-indan

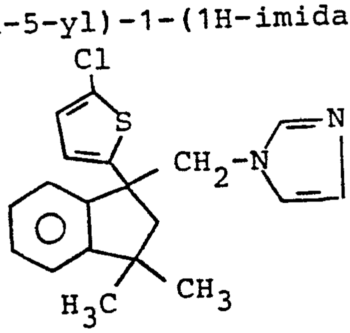

Zu einer auf -10°C gekühlten Suspension von 6,5 g (27 mmol) 1-(1H-Imidazol-1-ylmethyl)-3,3-dimethyl-indan-1-ol (Beispiel 36) in einem Gemisch aus 50 ml 1,2-Dichlorethan und 20 ml 2-Chlorthiophen gab man 8,0 g (60 mmol) Aluminiumtrichlorid. Nach Abklingen der exothermen Reaktion wurde 6h bei Raumtemperatur nachgerührt, auf Eisgegossen, mit 30 %iger Natronlauge alkalisch gestellt, die organische Phase abgetrennt, mit Wasser gewaschen, getrocknet und eingeengt. Chromatographie an Kieselgel ergab 5,7 g einer gelben, glasartigen Masse.
(62 % d. Th.)

Tabelle 1 enthält die vorstehend beschriebenen Endprodukte sowie weitere Verbindungen der Formel (I), die analog den angeführten Beispielen synthetisiert werden können

a) Verbindungen der Formel (I), für die W die zur Vervollständigung eines Benzolrings benötigten Atome bedeutet. Die Positionsangaben für $R^1$ und $R^2$ in Tabelle 1 beziehen sich auf die nachfolgend gezeichnete Formel; sie entsprechen nicht den üblichen Nomenklaturregeln

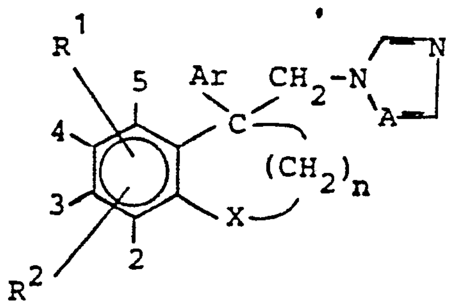

(I)

Tabelle 1

| Verb.-Nr. | A | X | n | Ar | $R^1$ | $R^2$ | Fp °C |
|---|---|---|---|---|---|---|---|
| 1 | CH | O | 2 | 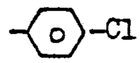 | 3-Cl | 4-Cl | 94° Zers.* |
| 2 | N | O | 2 | " | 3-Cl | H | Harz |
| 3 | CH | S | 2 | " | 3-Cl | H | 143-144 |
| 4 | CH | $CH_2$ | 2 | 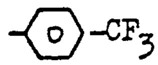 | H | H | Harz |
| 5 | N | $CH_2$ | 2 | 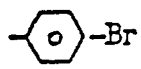 | H | H | 121 |
| 6 | CH | $CH_2$ | 2 | 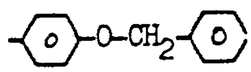 | H | H | 132-133 |
| 7 | CH | $CH_2$ | 2 | 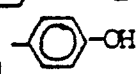 | H | H | 272-275 |
| 8 | CH | $CH_2$ | 2 | 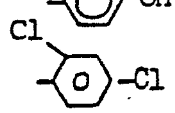 | 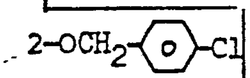 | H | 184-185 |
| 9 | CH | $CH_2$ | 2 | " | 2-OH | H | 245 Zers. |
| 10 | CH | $CH_2$ | 1 | 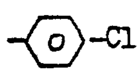 | H | H | Harz |
| 11 | CH | $CH_2$ | 2 | 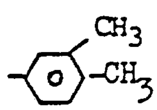 | H | H | 102-103 |
| 12 | CH | $CH_2$ | 3 | " | H | H | 158-160 |
| 13 | CH | $CH_2$ | 1 | 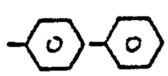 | H | H | 123-125 |
| 14 | CH | $CH_2$ | 2 | 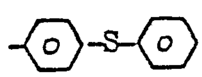 | H | H | 124 |
| 15 | CH | $CH_2$ | 2 | 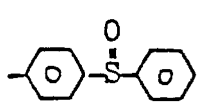 | H | H | Harz |
| 16 | CH | $CH_2$ | 2 | 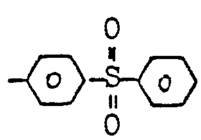 | H | H | 143-146 |

*)kristallisierte mit 1/3 Mol Diethylether

Tabelle 1 (Fortsetzung)

| Verb.-Nr. | A | X | n | Ar | $R^1$ | $R^2$ | Fp °C |
|---|---|---|---|---|---|---|---|
| 17 | CH | O | 2 | 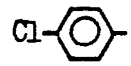 | 3-Cl | H | Harz |
| 18 | CH | $CH_2$ | 1 | 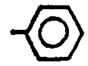 | H | H | |
| 18a | N | $CH_2$ | 1 | " | H | H | |
| 19 | CH | $CH_2$ | 2 | " | H | H | 147-149 |
| 19a | N | $CH_2$ | 2 | | H | H | |
| 20 | CH | $CH_2$ | 2 | 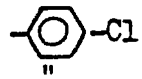 | H | H | 99-100 |
| 20a | N | $CH_2$ | 1 | " | H | H | |
| 21 | N | $CH_2$ | 2 | " | H | H | 127-128 |
| 22 | CH | $CH_2$ | 2 | " | 3-Cl | H | 147-148 |
| 23 | N | $CH_2$ | 2 | " | 3-Cl | H | 141-142 |
| 24 | CH | $CH_2$ | 2 | " | 4-Cl | H | 119-121 |
| 25 | CH | $CH_2$ | 2 | " | 3-Cl | 4-Cl | 145-146 |
| 26 | N | $CH_2$ | 2 | " | 3-Cl | 4-Cl | 168-169 |
| 27 | CH | $CH_2$ | 1 | 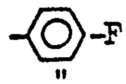 | H | H | |
| 27a | N | $CH_2$ | 1 | " | H | H | |
| 28 | CH | $CH_2$ | 1 | " | 3-F | H | |
| 29 | CH | $CH_2$ | 2 | " | H | H | 104-105 |
| 30 | CH | $CH_2$ | 2 | " | 3-F | H | 122-124 |
| 31 | CH | $CH_2$ | 1 | 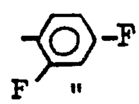 | H | H | 66-68 |
| 31a | N | $CH_2$ | 1 | " | H | H | |
| 32 | CH | $CH_2$ | 2 | " | H | H | 111-112 |
| 32a | N | $CH_2$ | 2 | " | H | H | |
| 33 | CH | $CH_2$ | 2 | 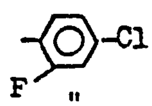 | H | H | 132-133 |
| 33a | N | $CH_2$ | 1 | " | H | H | |
| 34 | N | $CH_2$ | 2 | " | H | H | 102-104 |
| 35 | CH | $CH_2$ | 1 | 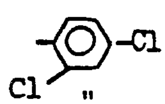 | H | H | Harz |
| 35a | N | $CH_2$ | 1 | " | H | H | |
| 36 | CH | $CH_2$ | 2 | " | H | H | 125-127 |
| 37 | N | $CH_2$ | 2 | " | H | H | 156-158 |
| 38 | CH | $CH_2$ | 2 | " | 2-Cl | H | Harz |

Tabelle 1 (Fortsetzung)

| Verb.-Nr. | A | X | n | Ar | $R^1$ | $R^2$ | Fp °C |
|---|---|---|---|---|---|---|---|
| 39 | CH | $CH_2$ | 2 | 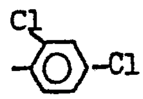 | 2-F | H | |
| 39a | N | $CH_2$ | 2 | | 2-F | H | |
| 40 | CH | $CH_2$ | 2 | " | 2-$CH_3$ | H | 141-143 |
| 41 | N | $CH_2$ | 2 | " | 2-$CH_3$ | H | 155-157 |
| 42 | CH | $CH_2$ | 2 | " | 2-$OCH_3$ | H | 154-155 |
| 42a | N | $CH_2$ | 2 | " | " | H | |
| 43 | CH | $CH_2$ | 2 | " | 2-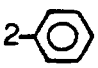 | H | 199-200 |
| 44 | CH | $CH_2$ | 1 | " | 2-O-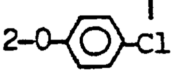 | H | |
| 45 | CH | $CH_2$ | 2 | " | 2-O-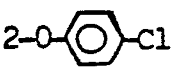 | H | 197-198 |
| 46 | CH | $CH_2$ | 2 | " | 4-Cl | H | 159-160 |
| 47 | CH | $CH_2$ | 2 | " | 3-F | H | |
| 47a | N | $CH_2$ | 2 | " | " | H | |
| 48 | N | $CH_2$ | 2 | 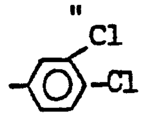 | H | H | 127-128 |
| 49 | CH | $CH_2$ | 2 | " | H | H | 56-57 Zers.* |
| 50 | CH | $CH_2$ | 2 | 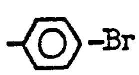 | H | H | 133 |
| 51 | CH | $CH_2$ | 2 | 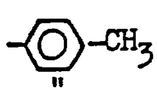 | H | H | 110-112 |
| 51a | N | $CH_2$ | 2 | " | H | H | |
| 52 | N | $CH_2$ | 2 | 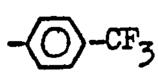 | H | H | 91-93 |
| 53 | CH | $CH_2$ | 1 | 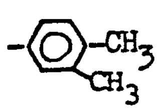 | H | H | 81-83 |
| 54 | CH | $CH_2$ | 2 | 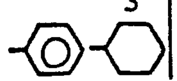 | H | H | 129-131 |
| 55 | CH | $CH_2$ | 2 | 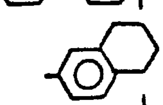 | H | H | Harz |
| 56 | CH | $CH_2$ | 3 | " | H | H | 139-141 |

*) kristallisierte mit 1/2 Mol Diethylether

0236913

Tabelle 1 (Fortsetzung)

| Verb.-Nr. | A | X | n | Ar | $R^1$ | $R^2$ | Fp. °C |
|---|---|---|---|---|---|---|---|
| 57 | CH | $CH_2$ | 2 | 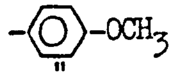 | H | H | 120-121 |
| 57a | N | $CH_2$ | 2 | " | H | H | |
| 58 | CH | $CH_2$ | 2 | 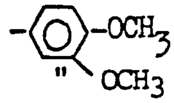 | H | H | 122-124 |
| 58a | N | $CH_2$ | 2 | " | H | H | |
| 59 | CH | $CH_2$ | 2 | 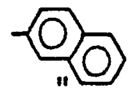 | H | H | 76-77 Zers.* |
| 59a | N | $CH_2$ | 2 | " | H | H | |
| 60 | CH | $CH_2$ | 2 | 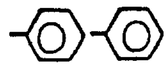 | H | H | 103-104 Zers.** |
| 60a | N | $CH_2$ | 2 | " | H | H | |
| 61 | CH | $CH_2$ | 2 | " | 3-F | H | |
| 62 | CH | $CH_2$ | 1 | " | 2-Cl | H | |
| 63 | CH | $CH_2$ | 1 | " | 3-Cl | H | |
| 64 | CH | $CH_2$ | 1 | " | 4-F | H | 121-122 |
| 65 | CH | $CH_2$ | 2 | 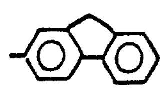 | H | H | Harz |
| 66 | CH | $CH_2$ | 1 | " | H | H | Harz |
| 67 | CH | $CH_2$ | 1 | 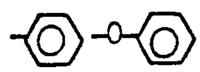 | H | H | Harz |
| 67a | N | $CH_2$ | 1 | " | H | H | |
| 68 | CH | $CH_2$ | 1 | " | 2-F | H | |
| 69 | CH | $CH_2$ | 1 | " | 2-$CH_3$ | H | |
| 70 | CH | $CH_2$ | 2 | " | H | H | 120-122 |
| 71 | CH | $CH_2$ | 1 | 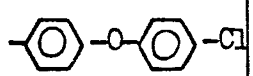 | H | H | 136-137 |
| 71a | N | $CH_2$ | 1 | " | H | H | |
| 72 | CH | $CH_2$ | 1 | " | 2-Cl | H | |
| 73 | CH | $CH_2$ | 2 | " | H | H | 138-139 |
| 73a | N | $CH_2$ | 2 | " | H | H | |
| 74 | CH | $CH_2$ | 1 | 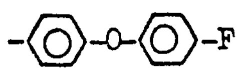 | H | H | |
| 74a | N | $CH_2$ | 1 | " | H | H | |

* kristallisierte mit 1/2 Mol Diethylether

** kristallisiert mit 1 Mol Methanol

Tabelle 1 (Fortsetzung)

| Verb.-Nr. | A | X | n | Ar | $R^1$ | $R^2$ | Fp °C |
|---|---|---|---|---|---|---|---|
| 75 | CH | $CH_2$ | 2 | 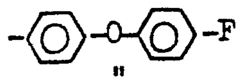 | H | H | 118-119 |
| 75a | N | $CH_2$ | 2 | " | H | H | |
| 76 | CH | $CH_2$ | 1 | 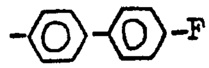 | H | H | 127-129 |
| 76a | N | $CH_2$ | 1 | " | H | H | |
| 77 | CH | $CH_2$ | 2 | " | H | H | |
| 77a | N | $CH_2$ | 2 | " | H | H | |
| 78 | CH | $CH_2$ | 2 | 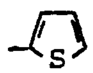 | H | H | 91-92 |
| 78a | N | $CH_2$ | 2 | " | H | H | |
| 79 | CH | $CH_2$ | 2 | 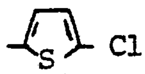 | H | H | 136-138 |
| 79a | N | $CH_2$ | 2 | " | H | H | |
| 80 | CH | $CH_2$ | 2 | 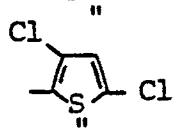 | H | H | 159-160 |
| 80a | N | $CH_2$ | 2 | " | H | H | 165-167 |
| 81 | CH | $CH_2$ | 1 | 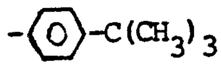 | H | H | Harz |
| 82 | CH | $CH_2$ | 2 | " | H | H | 121-122 |
| 83 | CH | $CH_2$ | 1 | 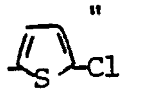 | 4-F | H | Harz |
| 84 | CH | $CH_2$ | 1 | 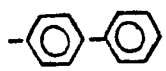 | 4-F | H | 121-122 |
| 85 | CH | $CH_2$ | 1 | " | 2-$CH_3$ | H | Harz |
| 86 | CH | $CH_2$ | 1 | 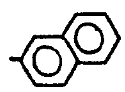 | H | H | 187-189* |
| 87 | CH | $CH_2$ | 1 | 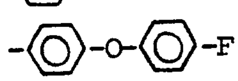 | H | H | 118-120 |
| 88 | CH | $CH_2$ | 2 | " | 3-Cl | 4-Cl | 93-96 |
| 89 | CH | $CH_2$ | 2 | " | 2-Cl | 4-Cl | Harz |
| 90 | CH | $CH_2$ | 2 | " | 2-F | 5-F | |
| 91 | CH | $CH_2$ | 2 | " | 3-Cl | 4-Cl | 120-122 |
| 92 | CH | $CH_2$ | 2 | " | 2-Cl | 4-Cl | Harz |
| 93 | CH | $CH_2$ | 2 | " | 2-F | 5-F | |
| 94 | CH | $CH_2$ | 2 | 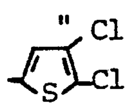 | H | H | 136-138 |
| 95 | CH | $CH_2$ | 2 | 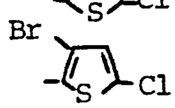 | " | " | 147-148 |
| 96 | CH | $CH_2$ | 2 | 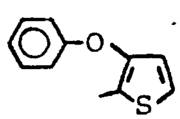 | H | H | 78-80 |

*) Hydrochlorid

Tabelle 1 (Fortsetzung)

| Verb.-Nr. | A | X | n | Ar | $R^1$ | $R^2$ | Fp °C |
|---|---|---|---|---|---|---|---|
| 97 | CH | $CH_2$ | 2 | 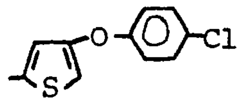 | H | H | |
| 98 | CH | $CH_2$ | 2 | 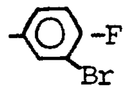 | H | H | |
| 99 | N | $CH_2$ | 2 | " | H | H | |
| 100 | CH | $CH_2$ | 2 | 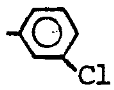 | H | H | |
| 101 | N | $CH_2$ | 2 | " | H | H | |
| 102 | CH | $CH_2$ | 2 | 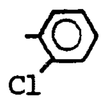 | H | H | |
| 103 | N | $CH_2$ | 2 | " | H | H | |
| 104 | CH | $CH_2$ | 2 | 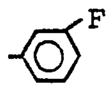 | H | H | |
| 105 | N | $CH_2$ | 2 | " | H | H | |
| 106 | CH | $CH_2$ | 2 | 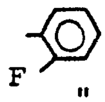 | H | H | |
| 107 | N | $CH_2$ | 2 | " | H | H | |
| 108 | CH | $CH_2$ | 2 | 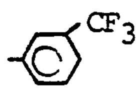 | H | H | |
| 109 | N | $CH_2$ | 2 | " | H | H | |
| 110 | CH | $CH_2$ | 2 | 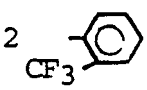 | H | H | |
| 111 | N | $CH_2$ | 2 | " | H | H | |
| 112 | CH | $CH_2$ | 2 | 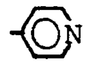 | H | H | |
| 113 | N | $CH_2$ | 2 | " | H | H | |
| 114 | CH | $CH_2$ | 2 | 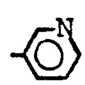 | H | H | |
| 115 | N | $CH_2$ | 2 | " | H | H | |
| 116 | CH | $CH_2$ | 2 | 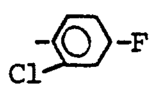 | H | H | |
| 117 | N | $CH_2$ | 2 | " | H | H | |
| 118 | CH | $CH_2$ | 2 | 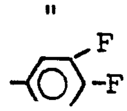 | H | H | |
| 119 | N | $CH_2$ | - | " | H | H | |
| 120 | CH | $CH_2$ | 2 | 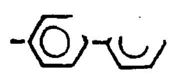 | 2-F | 5-F | |

0236913

Tabelle 1 (Fortsetzung)

| Verb.-Nr. | A | X | n | Ar | $R^1$ | $R^2$ | Fp °C |
|---|---|---|---|---|---|---|---|
| 121 | CH | $CH_2$ | 2 | 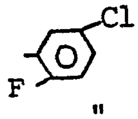 | H | H | |
| 122 | N | $CH_2$ | 2 | " | H | H | 103-105 |
| 123 | CH | $CH_2$ | 2 | 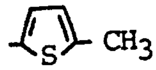 | H | H | |
| 124 | CH | $CH_2$ | 2 | 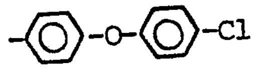 | 2-Cl | 4-Cl | |
| 125 | CH | $CH_2$ | 2 | " | 3-Cl | 4-Cl | |
| 126 | CH | $CH_2$ | 2 | " | 2-F | 5-F | |
| 127 | CH | $CH_2$ | 2 | 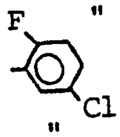 | H | H | |
| 128 | N | $CH_2$ | 2 | " | H | H | |
| 129 | CH | O | 3 | 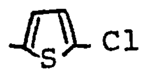 | 3-Cl | H | |
| 130 | N | O | 3 | " | 3-Cl | H | |
| 131 | CH | O | 3 | " | 4-Cl | H | |
| 132 | N | O | 3 | " | 4-Cl | H | |
| 133 | CH | O | 3 | " | 4-F | H | |
| 134 | N | O | 3 | " | 4-F | H | |
| 135 | CH | S | 3 | " | 4-Cl | H | |
| 136 | N | S | 3 | " | 4-Cl | H | |
| 137 | CH | O | 3 | 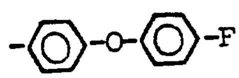 | 3-Cl | H | |
| 138 | N | O | 3 | " | 3-Cl | H | |
| 139 | CH | O | 3 | " | 4-Cl | H | |
| 140 | N | O | 3 | " | 4-Cl | H | |
| 141 | CH | O | 3 | " | 4-F | | |
| 142 | N | O | 3 | " | 4-F | H | |
| 143 | CH | S | 3 | " | 4-Cl | H | |
| 144 | N | S | 3 | " | 4-Cl | H | |
| 145 | CH | O | 3 | 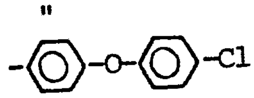 | 3-Cl | H | |
| 146 | N | O | 3 | " | 3-Cl | H | |
| 147 | CH | O | 3 | " | 4-Cl | H | |
| 148 | N | O | 3 | " | 4-Cl | H | |
| 149 | CH | O | 3 | " | 4-F | H | |
| 150 | N | O | 3 | " | 4-F | H | |
| 151 | CH | S | 3 | " | 4-Cl | H | |

Tabelle 1 (Fortsetzung)

| Verb.-Nr. | A | X | n | Ar | $R^1$ | $R^2$ | Fp °C |
|---|---|---|---|---|---|---|---|
| 152 | CH | O | 3 | 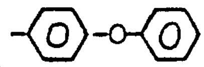 | 3-Cl | H | |
| 153 | N | O | 3 | " | 3-Cl | H | |
| 154 | CH | O | 3 | " | 4-Cl | H | |
| 155 | N | O | 3 | " | 4-Cl | H | |
| 156 | CH | O | 3 | " | 4-F | H | |
| 157 | N | O | 3 | " | 4-F | H | |
| 158 | CH | S | 3 | " | 4-Cl | H | |
| 159 | N | S | 3 | " | 4-Cl | H | |
| 160 | CH | $C(CH_3)_2$ | 2 | 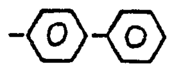 | H | H | Harz |

Verbindungen der Formel I mit $R_3$ # H

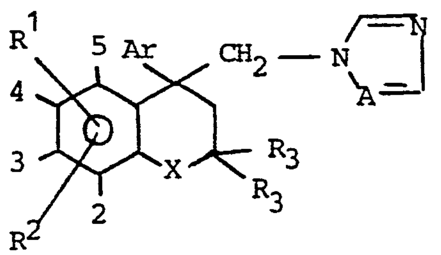

(I)

Tabelle 1a

| Verb.Nr. | A | X | Ar | $R^1$ | $R^2$ | $R^3$ | $R^3$ | Fp°C |
|---|---|---|---|---|---|---|---|---|
| 161 | CH | $CH_2$ | 4-Biphenylyl | H | H | $CH_3$ | $CH_3$ | Harz |
| 162 | CH | $CH_2$ | 2-Thienyl-5-Cl | H | H | $CH_3$ | $CH_3$ | Harz |
| 163 | CH | O | " | H | 3-Cl | $CH_3$ | $CH_3$ | |
| 164 | CH | O | 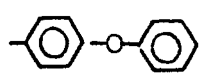 | H | 3-Cl | $CH_3$ | $CH_3$ | |
| 165 | CH | O | " | H | 3-Cl | $-(CH_2)_5-$ | | |
| 166 | CH | S | " | H | H | $CH_3$ | $CH_3$ | |

0236913

Verbindungen der Formel (I), für die W die zur Vervollständigung eines Naphthalin-Ringsystems nötigen Atome bedeutet:

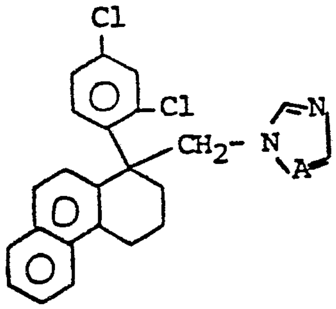

| Verb.-Nr. | A | Fp [°C] |
|---|---|---|
| 167 | CH | 214 |
| 168 | N | 177 |

Herstellung der Ausgangsprodukte

a) Herstellung der Verbindungen der Formel (IV)

Beispiel 17

2-(4-Chlorphenyl)-4-(3,4-dichlorphenoxy)-1-(1H-imidazol-1-yl)-butan-2-ol

(Ausgangsprodukt für Beispiel 1)

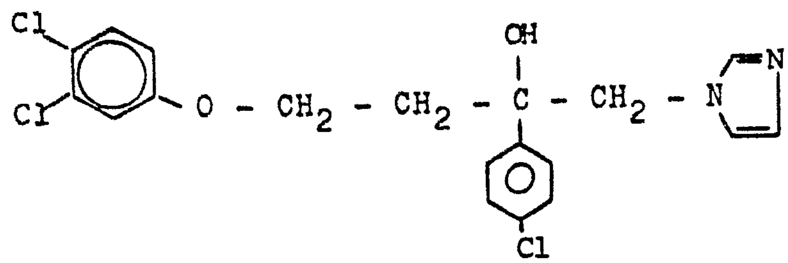

Zu einem Gemisch von 6,1 g (0,023 Mol) 2-(4-Chlorphenyl)-1-(1H-imidazol-1-yl)-butan-2,4-diol (Herstellung: EP-OS 150 036), 3,8 g (0,023 Mol) 3,4-Dichlorphenol und 6,5 g (0,025 Mol) Triphenylphosphin in 100 ml trockenen Tetrahydrofuran tropfteman unter Kühlung bei 20° eine Lösung von 4,5 g (0,025 Mol) Azodicarbonsäurediethylester in 10 ml trockenem Tetrahydrofuran. Nach Stehen über Nacht wurde eingeengt und das Rohgemisch an Kieselgel mit Ethylacetat/Methanol 9:1 chromatographiert. Das Produkt wurde als letzte Komponente von der Säule eluiert. Man erhielt ein farbloses Öl, das beim Verreiben mit Diethylether kristallisiert.

Ausbeute: 7,0 g (73,9 %), Fp: 139 - 140°C

Beispiel 18

2-(4-Chlorphenyl)-4-(3-chlorphenoxy)-1-(1,2,4-triazol-1-yl)-butan-2-ol

(Ausgangsprodukt für Beispiel 2)

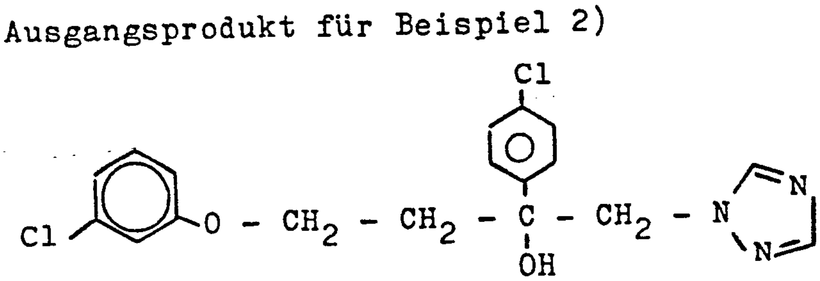

Analog Beispiel 17 wurden 6,9 g (0,026 Mol) 2-(4-Chlorphenyl)-1-(1,2,4-triazol-1-yl)-butan-2,4-diol (Beispiel 19)

3,5 g (0,027 Mol) 3-Chlorphenol, 7,0 g (0,027 Mol) Triphenylphosphin und 5,0 (0,029 Mol) Azodicarbonsäurediethylester umgesetzt. Nach Chromatographie an Kieselgel (Produkt wird als vorletzte Fraktion eluiert) wurden 4,0 g (40,1 % d.Th.) harzartiges Produkt isoliert.

Beispiel 19

2-(4-Chlorphenyl)-1-(1,2,4-triazol-1-yl)-butan-2,4-diol

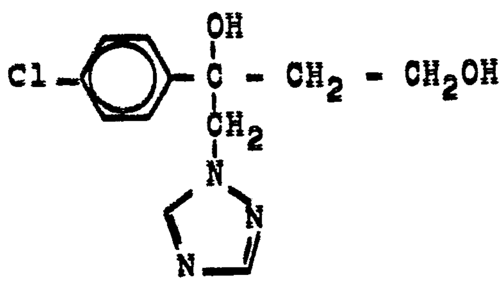

Zu einer Lösung von 1,5 g Lithiumaluminiumhydrid in 50 ml trockenem Tetrahydrofuran tropfte man unter Rühren und Kühlung eine Lösung von 8,9 g (0,029 Mol) 3-(4-Chlorphenyl)-3-hydroxy-4-(1,2,4-triazol-1-yl)-buttersäure-ethylester (Beispiel 20). Man rührte 6 Stunden bei Raumtemperatur nach, zersetzte unter Kühlung vorsichtig mit Wasser, saugte vom anorganischen Material ab, trocknete und engte ein. Man erhielt ein farbloses Öl, das beim Verreiben mit Diethylether kristallisierte.

Ausbeute: 7,0 g (89,8 % d.Th.)

Fp.: 108 - 109°C

Beispiel 20

3-(4-Chlorphenyl)-3-hydroxy-4-(1,2,4-triazol-1-yl)-buttersäure-ethylester

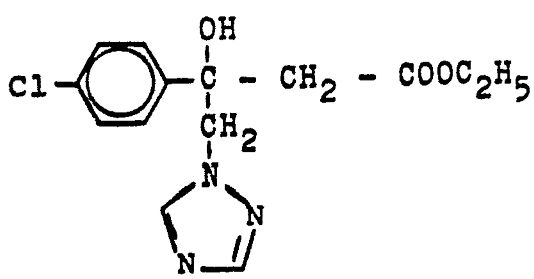

Man legte 100 ml einer 1 M-Lösung von Lithium-bis-(tri-

methylsilyl)-amid in Tetrahydrofuran vor und tropfte bei -78°C 9,7 ml trockenen Essigsäureäthylester zu. Nach 15-minütigem Rühren bei der gleichen Temperatur verdünnte man langsam mit 100 ml trockenem Tetrahydrofuran und gab dann bei -78° portionsweise 22,1 g (0,1 Mol) fein pulverisiertes 4-Chlorphenyl-(1,2,4-triazol-1-yl)-methyl-keton zu. Man rührte 2 Stunden bei -78°, goß in Wasser, schüttelte mit Methylenchlorid aus, trocknete die organische Phase und engte ein. Das Rohprodukt wurde an Kieselgel mit Ethylacetat chromatographiert. Man erhielt 12,1 g farblose Kristalle (39,5 % d.Th.), Fp.: 93 - 94°C

Beispiel 21
2-(4-Chlorphenyl)-1-(1H-imidazol-1-yl)-4-(3-chlorphenylthio)-butan-2-ol
(Ausgangsprodukt für Beispiel 3)

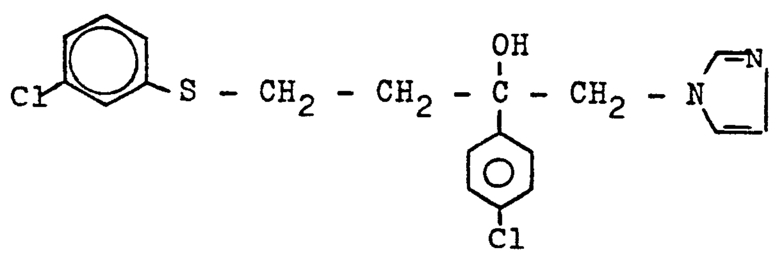

Analog Beispiel 17 wurden 12,0 g (0,045 Mol) 2-(4-Chlorphenyl)-1-(1H-imidazol-1-yl)-butan-2,4-diol (EP-OS 150 036) 7,0 g (0,048 Mol) 3-Chlorthiophenol und 13,5 g (0.051 Mol) Triphenylphosphin mit 8,9 g (0,051 Mol) Azodicarbonsäurediethylester umgesetzt. Chromatographie am Kieselgel (das Produkt wurde als letzte Fraktion von der Säule eluiert) ergab 6,5 g farbloses Harz (36,8 % d.Th.).

0236913

Beispiel 22

1-(1H-Imidazol-1-yl)-5-phenyl-2-(4-trifluormethylphenyl)-pentan-2-ol

(Ausgangsprodukt für Beispiel 4)

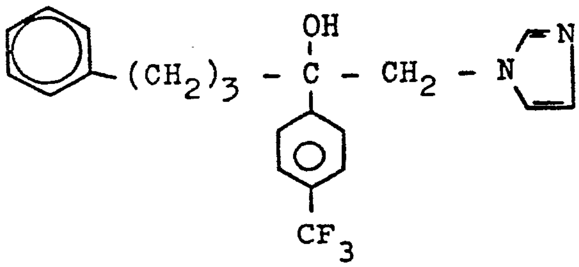

Zu einer Suspension von 1,8 g Natriumhydrid (80 % in Öl) (0,C5 Mol) in 100 ml trockenem Dimethylsulfoxid gab man unter Kühlung bei einer Temperatur von 15 - 20°C portionsweise 13,2 g (0,065 Mol) Trimethylsulfoxoniumjodid und rührte bis zum Ende der Gasentwicklung. Nun gab man eine Lösung von 14,6 g (0,05 Mol) 1-Phenyl-3-(4-trifluormethylbenzoyl)-propan (Beispiel 23) in 100 ml trockenem Dimethylsulfoxid zu. Nach Abkühlen der schwach exothermen Reaktion wurde auf 50°C erwärmt. Nach einer Stunde gab man 4,5 g (0,C5 Mol) Imidazol-Natriumsalz zu der Mischung, rührte eine weitere Stunde bei 50°C und 3 Stunden bei 80°C. Zur Aufarbeitung wurde auf Eis gegossen, mit Methylenchlorid aufgenommen, die organische Phase getrocknet und eingeengt. Zur Reinigung wurde an Kieselgel chromatographiert (Ethylacetat/Methanol 9:1). Man erhielt ein farbloses Öl, das beim Verreiben mit Diethylether kristallisierte.

Ausbeute: 12,6 g (67,3 % d.Th.)

Fp.: 147 - 148°C

Beispiel 23

1-Phenyl-3-(trifluormethylbenzoyl)-propan

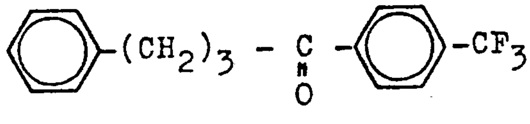

Zu einer aus 5,4 g (0,22 Mol) Magnesiumspänen und 50,0 g

(0,22 Mol) 4-Brombenzotrifluorid in 100 ml trockenem Diethylether bereiteten Grignardlösung tropfte man eine Lösung von 29,0 g (0,20 Mol) 4-Phenylbutyronitril in 50 ml trockenem Diethylether. Das Reaktionsgemisch kam zum Sieden und wurde weitere 3 Stunden unter Rückfluß gerührt. Man ließ abkühlen, goß auf Eis, stellt mit 10 %iger Schwefelsäure stark sauer und rührte das Gemisch 30 Minuten. Anschließend wurde die organische Phase abgetrennt, die Wasserphase nochmals mit Ether ausgeschüttelt und die vereinigten organischen Phasen getrocknet und eingeengt. Der ölige Rückstand wurde mit Methanol verrührt. Beim Abkühlen und Anreiben kristallisierte 30,8 g (52,7 % d.Th.) farbloses Produkt aus.
Fp.: 56 - 58°C.

Beispiel 24
2-(4-Bromphenyl)-5-phenyl-1-(1,2,4-triazol-1-yl)-pentan-2-ol
(Ausgangsprodukt für Beispiel 5)

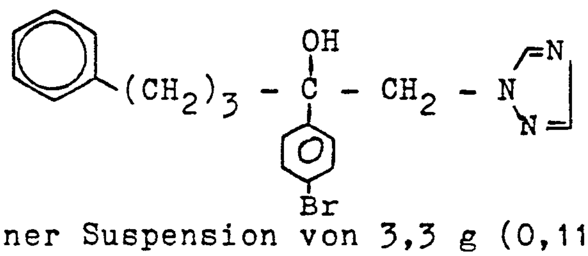

Zu einer Suspension von 3,3 g (0,11 Mol) Natriumhydrid (80 %ig in Öl) in 150 ml trockenem Dimethylsulfoxid gab man unter Kühlung bei 15 - 20°C portionsweise 24,2 g (0,11 Mol) Trimethylsulfoxoniumjodid und rührte bis zum Ende der Gasentwicklung. Nun gab man eine Lösung von 26,6 g (0,09 Mol) 1-(4-Brombenzoyl)-3-phenyl-propan (Beispiel 25) in 100 ml Dimethylsulfoxid/Tetrahydrofuran 1:1 zu. Man rührte eine Stunde bei Raumtemperatur und eine weitere Stunde bei 50°C. Anschließend gab man 9,0 g (0,10 Mol) 1,2,4-Triazol-Natriumsalz zu und rührte weitere 3 Stunden bei 50°C. Zur Aufarbeitung wurde auf Wasser gegossen, mit Methylenchlorid ausgerührt, die organische Phase getrocknet und eingeengt. Nach Chromatographie an

Kieselgel mit Ethylacetat erhielt man ein farbloses Öl, das beim Verreiben mit Diethylether kristallisierte.
Ausbeute: 16,8 g (48,3 % d.Th.)
Fp.: 90°C

Beispiel 25
1-(4-Brombenzoyl)-3-phenyl-propan

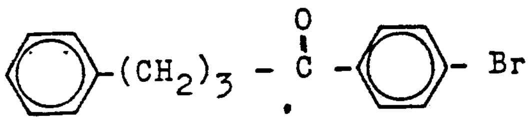

Zu einer aus 8,8 g (0,36 Mol) Magnesiumspänen und 69,8 g (0,35 Mol) 1-Brom-3-phenylpropan in 250 ml trockenem Diethylether zubereiteten Grignard-Lösung tropfte man eine Lösung von 60,0 g (0,33 Mol) 4-Brom-benzonitril in 250 ml trockenem Diethylether. Nach Abklingen der exothermen Reaktion wurde 6 h unter Rückfluß gerührt. Nach Abkühlen wurde auf Eis gegossen, mit 10 %iger Schwefelsäure sauer gestellt und das Gemisch eine Stunde nachgerührt. Man rührte mehrere Male mit Methylenchlorid aus, trocknete die vereinigten organischen Phasen und engte ein. Der Rückstand wurde aus Methanol umkristallisiert.
Ausbeute: 53,2 g farblose Kristalle (53,2 %)
Fp.: 60 - 61°C

Beispiel 26
2-(4-Chlorphenyl)-1-(1H-imidazol-1-yl)-4-phenyl-butan-2-ol
(Ausgangsprodukt für Beispiel 10)

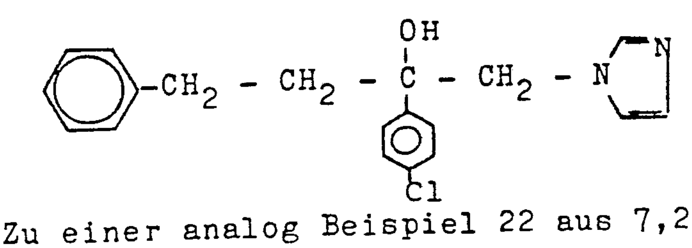

Zu einer analog Beispiel 22 aus 7,2 g (0,24 Mol) Natriumhydrid (80 %ig in Öl) und 57,2 g (0,26 Mol) Trimethylsulfoxoniumjodid in 250 ml trockenem Dimethylsulfoxid hergestellten Lösung von Dimethylsulfoxoniummethylid gab man eine Lösung von 49,0 g (0,20 Mol) 1-(4-Chlorbenzoyl)-2-phenyl-ethan (Beispiel 27) in 150 ml trockenem Dimethylsulfoxid. Man rührte 3 h bei Raumtemperatur und 1 h bei 50°C. Nach Zugabe von 18,9 g (0,21 Mol) Imidazol-Natriumsalz wurde weitere 5 h bei 80°C gerührt. Nach Abkühlen wurde auf Eis gegossen und mit 400 ml Methylenchlorid verrührt. Ein Teil des gewünschten Produkts blieb ungelöst und wurde abgesaugt. Die Methylenchloridphase wurde getrocknet und eingeengt. Das verbleibende Öl wurde mit Diethylether verrührt wobei weiteres Produkt auskristallisierte.

Gesamtausbeute: 44,7 g (68,4 % d.Th.)

Fp.: 186 - 188°C

Beispiel 27

1-(4-Chlorbenzoyl)-2-phenyl-ethan

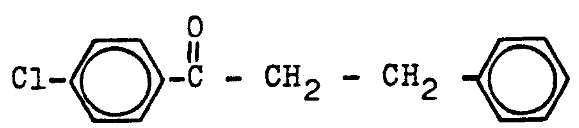

Man löste 75,0 g (0,31 Mol) 3-(4-Chlorphenyl)-1-phenyl-1-propen-3-on (hergestellt durch Aldolkondensation aus 4-Chloracetophenon und Benzaldehyd) in 200 ml Tetrahydrofuran und hydrierte nach Zugabe von 5 g Palladium/Kohlekatalysator (5 % Pd) unter Normalbedingungen. Nach Ende der Wasserstoffaufnahme wurde vom Katalysator abfiltriert und eingeengt. Der Rückstand wurde aus Ethanol umkristallisiert.

Ausbeute: 57,0 g (75,4 % d.Th.)

Fp.: 76 - 78°C

0236913

Beispiel 28

5-[2-(4-Chlorphenoxy)phenyl]-2-(2,4-dichlorphenyl)-1-(1H-imidazol-1-yl)-pentan-2-ol

(Ausgangsprodukt für Verbindung Nr. 45, Tabelle 1)

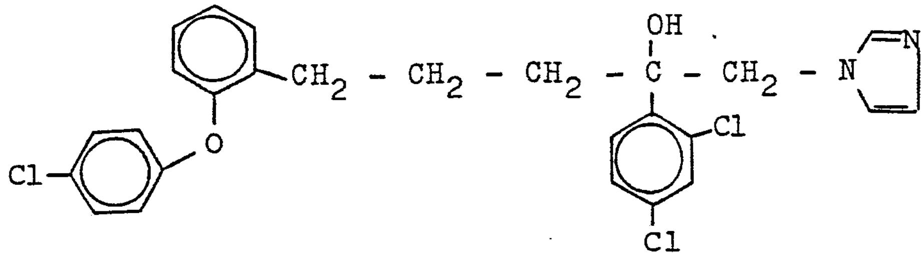

Zu einer analog Beispiel 22 aus 3,6 g (0,12 Mol) Natriumhydrid (80 %ig in Öl) und 28,6 g (0,13 Mol) Trimethylsulfoxoniumjodid in 150 ml trockenem Dimethylsulfoxid hergestellten Lösung von Dimethylsulfoxoniummethylid gab man eine Lösung 41,0 g (0,10 Mol) 1-[2-(4-Chlorphenoxy)-phenyl]-3-(2,4-dichlorbenzoyl)-propan (Beispiel 29) in 150 ml trockenem Dimethylsulfoxid/Tetrahydrofuran (1:1). Man rührte 3 h bei Raumtemperatur und eine Stunde bei 50°C. Bei dieser Temperatur gab man 10,0 g (0,11 Mol) Imidazol-Natriumsalz zu, rührte 4 h bei 50°C und 1 h bei 80°C. Die Aufarbeitung erfolgte analog Beispiel 22. Chromatographie an Kieselgel ergab 17,0 g (34,7 % d.Th.) farblose Kristalle Fp.: 159 - 160°C

Beispiel 29

1-[2-(4-Chlorphenoxy)phenyl]-3-(2,4-dichlorbenzoyl)-propan

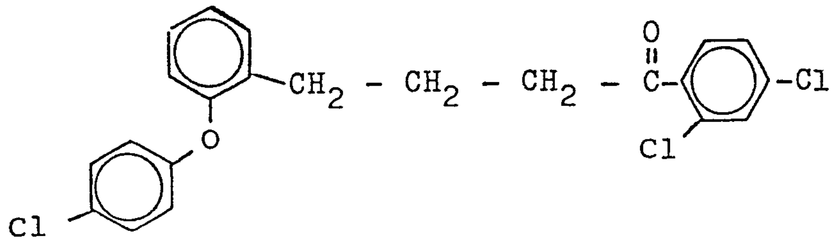

Eine Lösung von 42,5 g (0,10 Mol) E-1-[2-(4-Chlorphenoxy)-phenyl]-2-(2,4-dichlorbenzoyl)-cyclopropan (Beispiel 30) in 250 ml Tetrahydrofuran wurde nach Zugabe von 5,0 g

0236913

Palladium/Kohle-Katalysator (5 % Pd) unter Normalbedingungen hydriert. Nach Abfiltrieren vom Katalysator und Einengen erhielt man 41,0 g (96,0 % d.Th.) harzartiges Produkt, das ohne weitere Reinigung umgesetzt wurde.

Beispiel 30

E-1-[2-(4-Chlorphenoxy)phenyl]-2-(2,4-dichlorbenzoyl)-cyclopropan

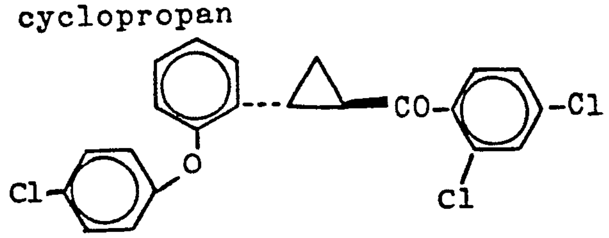

Zu einer analog Beispiel 22 aus 5,3 g (0,18 Mol) Natriumhydrid (80 %ig in Öl) und 42,5 g (0,19 Mol) Trimethylsulfoxoniumjodid in 250 ml trockenem Dimethylsulfoxid hergestellten Lösung von Dimethylsulfoxoniummethylid gab man unter Kühlung eine Lösung von 60,0 g (0,15 Mol) 1-[2-(4-Chlorphenoxy)phenyl]-3-(2,4-dichlorphenyl)-1-propen-3-on Beispiel 31). Man rührte 3 h bei Raumtemperatur, goß auf 1,5 l Wasser und saugte den ausgefallenen Feststoff ab. Zur Reinigung wurde mit 200 ml Ethanol aufgekocht.

Ausbeute: 42,5 g farblose Kristalle (67,8 % d.Th.)

Fp.: 74 - 75°C

Beispiel 31

1-[2-(4-Chlorphenoxy)phenyl]-3-(2,4-dichlorphenyl)-1-propen-3-on

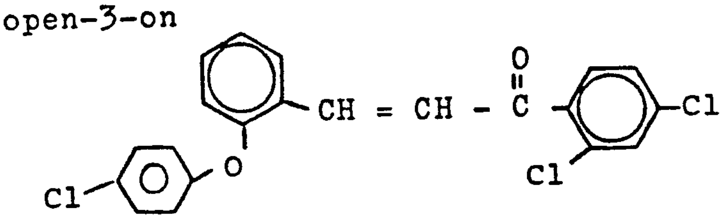

Zu einer Lösung von 56,7 g (0,3 Mol) 2,4-Dichloracetophenon und 68,0 g (0,3 Mol) 2-(4-Chlorphenoxy)-benzaldehyd (Beispiel 32) in 400 ml Methanol gab man unter Rühren 6 ml 15 %ige Kalilauge. Man rührte 8 h bei Raumtemperatur,

neutralisierte mit Eisessig und goß auf Wasser. Man rührte mit Methylenchlorid aus, trocknete die organische Phase und engte ein. Zur Reinigung wurde an Kieselgel chromatographiert (Hexan/Methylenchlorid 1:1). Man erhielt 102,0 g (84,2 % d.Th.) eines gelben Öls.

Beispiel 32

2-(4-Chlorphenoxy)-benzaldehyd

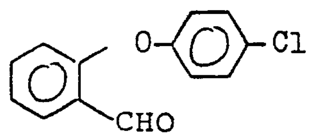

In eine Lösung von 154,3g (1,2 Mol) 4-Chlorphenol in 1 l trockenem Dimethylformamid wurde portionsweise unter Kühlung 33,0 g (1,1 Mol) Natriumhydrid (80 %ig in Öl) eingetragen. Nach Ende der Gasentwicklung gab man 124,1 g (1,0 Mol) 2-Fluorbenzaldehyd zu und rührte 6 h bei 80°C. Nach Abkühlen wurde auf Eis gegossen und das ausgefallene Produkt abgesaugt. Man löst nochmals in Methylenchlorid, schüttelte mit Wasser, trocknete und engte ein. Umkristallisation aus Cyclohexan ergab 149,3 g farblose Kristalle (64,2 % d.Th.) Fp: 61 - 62°C

In Tabelle 2 sind die vorstehend beschriebenen, sowie weitere Verbindungen der Formel IV aufgeführt, die analog den vorstehenden Beispielen synthetisiert werden können

a) Verbindungen der Formel (IV), für die W die zur Vervollständigung eines Benzol-Ringsystems benötigten Atome bedeutet.

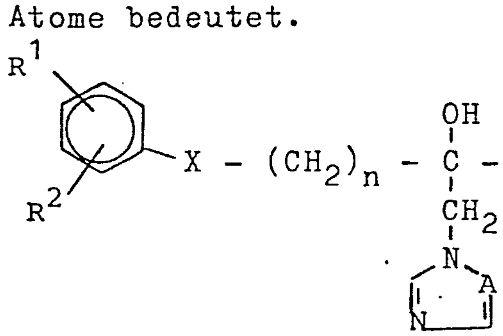

(IV)

Tabelle 2

| Verb.-Nr. | A | X | n | Ar | $R^1$ | $R^2$ | Fp °C |
|---|---|---|---|---|---|---|---|
| 1 | CH | O | 2 | 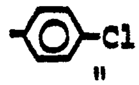 | 3-Cl | H | 77-79 |
| 2 | N | O | 2 | " | 3-Cl | H | Harz |
| 3 | CH | O | 2 | " | 3-Cl | 4-Cl | 139-140 |
| 4 | CH | S | 2 | | 3-Cl | H | Harz |
| 5 | CH | $CH_2$ | 2 | " | H | H | 164-165 |
| 6 | N | $CH_2$ | 2 | " | H | H | 90 |
| 7 | CH | $CH_2$ | 2 | " | 3-Cl | H | 148-149 |
| 8 | N | $CH_2$ | 2 | " | 3-Cl | H | Harz |
| 9 | CH | $CH_2$ | 2 | " | 4-Cl | H | 185-187 |
| 10 | CH | $CH_2$ | 2 | " | 3-Cl | 4-Cl | 182-183 |
| 11 | N | $CH_2$ | 2 | " | 3-Cl | 4-Cl | 111-112 |
| 12 | CH | $CH_2$ | 1 | " | H | H | 186-188 |
| 13 | N | $CH_2$ | 1 | " | H | H | 127-128 |
| 14 | CH | $CH_2$ | 3 | " | H | H | 121-125 |
| 15 | CH | $CH_2$ | 2 | 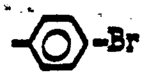 | H | H | 144-145 |
| 16 | N | $CH_2$ | 2 | " | H | H | 90 |
| 17 | CH | $CH_2$ | 2 | 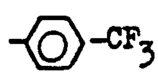 | H | H | 147-148 |
| 18 | N | $CH_2$ | 2 | " | H | H | 93-94 |
| 19 | CH | $CH_2$ | 2 | 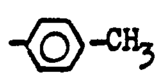 | H | H | 164-166 |
| 20 | CH | $CH_2$ | 2 | 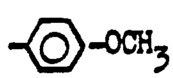 | H | H | 160-161 |
| 21 | CH | $CH_2$ | 2 | 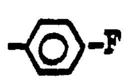 | 3-F | H | 177-178 |
| 22 | CH | $CH_2$ | 2 | 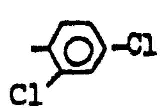 | H | H | 168-169 |

Tabelle 2 (Fortsetzung)

| Verb.-Nr. | A | X | n | Ar | $R^1$ | $R^2$ | Fp °C |
|---|---|---|---|---|---|---|---|
| 23 | N | $CH_2$ | 2 | 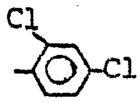 | H | H | 126 |
| 24 | CH | $CH_2$ | 2 | " | 4-Cl | H | 153-155 |
| 25 | CH | $CH_2$ | 2 | " | 2-Cl | | 154-157 |
| 26 | CH | $CH_2$ | 2 | " | $2-OCH_3$ | H | 140-141 |
| 27 | CH | $CH_2$ | 2 | " | $2-CH_3$ | H | 164-165 |
| 28 | N | $CH_2$ | 2 | " | $2-CH_3$ | H | 120-122 |
| 29 | CH | $CH_2$ | 2 | " | 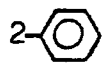 | H | Harz |
| 30 | CH | $CH_2$ | 2 | " | 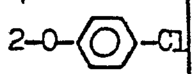 | H | 159-160 |
| 31 | CH | $CH_2$ | 2 | 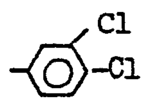 | H | H | 177-179 |
| 32 | N | $CH_2$ | 2 | " | H | H | 93-95 |
| 33 | CH | $CH_2$ | 2 | 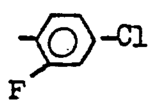 | H | H | 144-146 |
| 34 | N | $CH_2$ | 2 | " | H | H | 109-111 |
| 35 | CH | $CH_2$ | 2 | 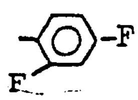 | H | H | 134-136 |
| 36 | CH | $CH_2$ | 1 | 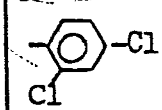 | H | H | 156-157 |
| 37 | CH | $CH_2$ | 2 | $-C_6H_5$ | H | H | 147-149 |
| 38 | CH | $CH_2$ | 2 | $-C_6H_4-4-F$ | H | H | 176-178 |
| 39 | CH | $CH_2$ | 2 | -(2)Thienyl | H | H | 150-152 |

b) Verbindungen der Formel (IV), für die W die zur Vervollständigung eines Naphthalin-Ringsystems benötigten Atome bedeutet

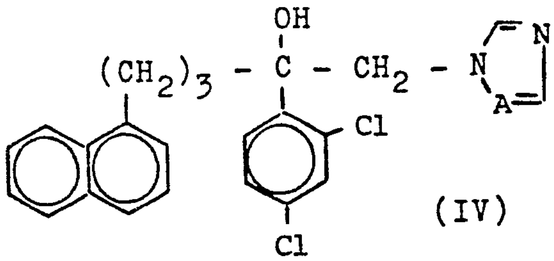

(IV)

| Verb.Nr. | A | Fp[°C] |
|---|---|---|
| 36 | CH | 102-103 |
| 37 | N | 97-98 |

Herstellung der Ausgangsprodukte der Formel (II)

Beispiel 33

1-Hydroxy-1-(1H-imidazol-1-ylmethyl)-tetralin

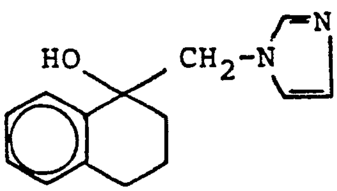

Zu einer analog Beispiel 22 aus 19,6 g (0,65 Mol) Natriumhydrid (80 %ig in Öl) und 157,0 g (0,71 Mol) Trimethylsulfoxoniumjodid in 750 ml trockenem Dimethylsulfoxid hergestellten Lösung von Dimethylsulfoxoniummethylid gab man eine Lösung von 79,6 g (0,55 Mol) 1-Tetralon in 150 ml trockenem Dimethylsulfoxid/Tetrahydrofuran 1:1 und rührte 5 h bei Raumtemperatur nach. In der Zwischenzeit stellte man durch Zugabe von 16,4 g (0,55 Mol) Natriumhydrid (80 %ig in Öl) zu einer Lösung von 74,1 g (1,1 Mol) Imidazol in 300 ml trockenem Dimethylsulfoxid eine äquimolare Lösung von Imidazol/Imidazol-Natriumsalz her und goß diese anschließend zu der Epoxid-Lösung. Nach Stehen über Nacht wurde 2 h bei 80°C gerührt und nach Abkühlen auf Eis gegossen. Man rührte mit 1 l Methylenchlorid aus und rührte die Methylenchloridphase zweimal mit je 500 ml 2n-Salzsäure aus. Die vereinigten Säurephasen wurden mit 50 %iger Natronlauge alkalisch gestellt und zwei Mal mit je 500 ml Methylenchlorid ausgerührt. Die vereinigten organischen Phasen wurden getrocknet und eingeengt. Das verbleibende Öl wurde mit Ethylacetat/Diethylether verrieben. Es kristallisierten 75,2 g (60,4 % d.Th.) farblose Kristalle aus.
Fp.: 152-154°C

Beispiel 34

4-Methyl-1-(1H-imidazol-1-ylmethyl)-indan-1-ol

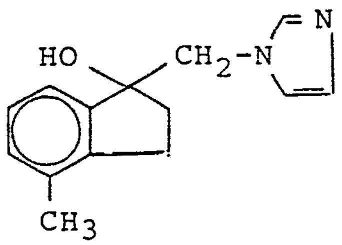

Zu einer analog Beispiel 22 aus 6,4 g (0,21 Mol) Natriumhydrid (80 %ig in Öl) und 50,9 g (0,23 Mol) Trimethylsulfoxoniumjodid in 250 ml trockenem Dimethylsulfoxid hergestellten Lösung von Dimethylsulfoxoniummethylid gab man eine Lösung von 26,0 g (0,18 Mol) 4-Methyl-indan-1-on (Beispiel 35) in 170 ml trockenem Tetrahydrofuran. Man rührte 1 h bei Raumtemperatur und 1 h bei 50°C. Bei dieser Temperatur wurden 16,2 g (0,18 Mol) Imidazol-Natriumsalz und 12,2 g (0,18 Mol) Imidazol zugegeben. Man rührte eine weitere Stunde bei 50°C und 3 h bei 80°C. Man arbeitete analog Beispiel 33 auf. Man erhielt 17,8 g farblose Kristalle (43,8 % d.Th.). Fp.: 155-157°C

Beispiel 35

4-Methyl-indan-1-on

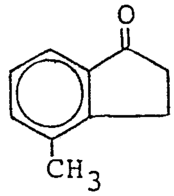

a) 2-Methyl-zimtsäure wurde an Palladium/Kohle hydriert,

und die so erhaltene 3-(2-Methylphenyl)-propionsäure (Fp: 97-98°C) mit Thionylchlorid ins Säurechlorid überführt (Sdp: 125°C/20 mm).

b) Zu einer Suspension von 40,0 g (0,3 Mol) Aluminiumtrichlorid in einem Gemisch aus 200 ml Schwefelkohlenstoff und 100 ml 1,2-Dichlorethan tropfte man unter heftigem Rühren bei einer Temperatur von 20°C 50,6 g (0,28 Mol) 3-(2-Methylphenyl)-propionsäurechlorid. Man rührte 6 h bei Raumtemperatur nach, goß auf Eis und stellte mit konz. Salzsäure stark sauer. Die organische Phase wurde getrocknet und eingeengt. Das Rohprodukt wurde aus Ethanol umkristallisiert. Man erhielt 26,8 g (66,2 % d.Th.) gelbe Kristalle.
Fp.: 103-104°C

Beispiel 36

1-(1H-Imidazol-1-ylmethyl)-3,3-dimethyl-indan-1-ol

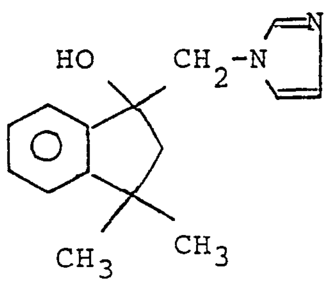

Zu einer aus 6,5 g Natriumhydrid (80 %ig in Öl) und 50,0 g (0,23 Mol) Trimethylsulfoxoniumjodid in 300 ml trockenem Dimethylsulfoxid hergestellten Lösung von Dimethylsulfoxoniummethylid gab man eine Lösung von 32,0 g (0,20 Mol) 3,3-Dimethylindan-1-on, hergestellt aus 3-Methyl-3-phenylbuttersäure (J.Amer.Chem.Soc. 76, 1911 (1954)) durch Überführung ins Säurechlorid und Cyclisierung mit Aluminiumchlorid , in 100 ml trockenem Dimethylsulfoxid. Man rühte 2h bei Raumtemperatur und 2h bei 50°C. Nach Zugabe von 14,0 g (0,2 Mol) Imidazol und 18,0 g (0,2 Mol) Imidazol-Natriumsalz wurde weitere 2h bei 50°C

und 2 h bei 70°C gerührt. Nach Abkühlen wurde auf Eis gegossen, mit Trockeneis neutralisiert und das ausgefallene Produkt abgesaugt. Zur Reinigung wurde das Rohprodukt mit Essigester erhitzt. Man erhielt 20,0 g farblose Kristalle (41 % d.Th.)

Fp. 166-168°C

In Tabelle 3 sind weitere Verbindungen der Formel (II) aufgeführt (für die W die zur Vervollständigung eines Benzol-Ringsystems benötigten Atome bedeutet), die sich analog den vorstehenden Beispielen synthetisieren lassen. Die Positionsangaben für $R^1$ und $R^2$ beziehen sich auf die nachfolgend gezeichnete Formel, sie entsprechen nicht den üblichen Nomenklaturregeln.

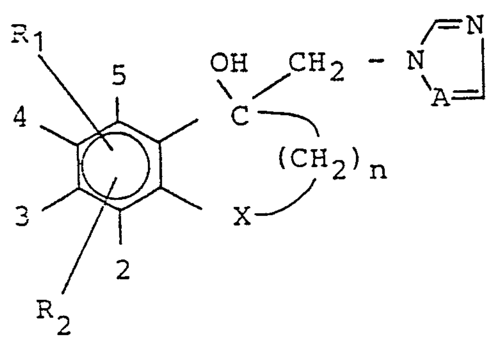

(II)

Tabelle 3

| Verb.-Nr. | A | X | n | $R^1$ | $R^2$ | Fp °C |
|---|---|---|---|---|---|---|
| 1 | CH | $CH_2$ | 1 | H | H | 129-130 |
| 2 | CH | S | 2 | H | H | 162-163 |
| 3 | CH | $CH_2$ | 3 | H | H | 164-165 |
| 4 | N | $CH_2$ | 3 | H | H | 108-109 |
| 5 | CH | $CH_2$ | 1 | 3-Cl | 4-Cl | 164-165 |
| 6 | CH | $CH_2$ | 1 | 3-F | H | 148-149 |
| 7 | CH | $CH_2$ | 1 | 2-F | H | 144-146 |
| 8 | CH | $CH_2$ | 1 | 4-F | H | 175-176 |
| 9 | CH | $CH_2$ | 1 | 3-$CH_3$ | H | 149-151 |
| 10 | CH | $CH_2$ | 1 | 4-$CH_3$ | H | 152-154 |
| 11 | CH | $CH_2$ | 1 | 3-O-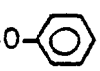 | H | 136-138 |
| 12 | CH | $CH_2$ | 1 | 4-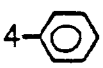 | H | 172 (Hydrochlorid) |
| 13 | CH | $CH_2$ | 1 | 2-$CH_3$ | H | 155-157 |
| 14 | CH | $CH_2$ | 1 | 5-$CH_3$ | H | 149-150 |
| 15 | CH | $CH_2$ | 2 | 2-$CH_3$ | 4-$CH_3$ | 190-191 |
| 16 | CH | $CH_2$ | 2 | 2-Cl | 4-Cl | 163-165 |
| 17 | CH | $CH_2$ | 2 | 3-Cl | 4-Cl | 159-160 |
| 18 | CH | $CH_2$ | 2 | 2-F | 5-F | |
| 19 | CH | $CH_2$ | 1 | 2-O-$C_6H_5$ | H | Harz |
| 20 | CH | $CH_2$ | 2 | 4-O-$C_6H_5$ | H | 119-121 |
| 21 | CH | $CH_2$ | 2 | 3-O-$C_6H_5$ | H | 138-139 |
| 22 | CH | $CH_2$ | 2 | 4-S-$C_6H_5$ | H | 131-133 |
| 23 | CH | $CH_2$ | 2 | 3-F | H | 162-165 |
| 24 | CH | $CH_2$ | 2 | 3-$OCH_3$ | H | 158-159 |
| 25 | CH | $CH_2$ | 2 | 4-$OCH_3$ | H | 161-162 |
| 26 | CH | $CH_2$ | 2 | 1-$OCH_3$ | H | 143-145 |
| 27 | CH | O | 3 | H | H | |
| 28 | N | O | 3 | H | H | |

Fortsetzung Tabelle 3

| Verb.-Nr. | A | X | n | $R^1$ | $R^2$ | Fp °C |
|---|---|---|---|---|---|---|
| 29 | CH | O | 3 | 4-Cl | H | |
| 30 | N | O | 3 | 4-Cl | H | |
| 31 | CH | O | 3 | 3-Cl | H | |
| 32 | N | O | 3 | 3-Cl | H | |
| 33 | CH | O | 3 | 4-F | H | |
| 34 | N | O | 3 | 4-F | H | |
| 35 | CH | S | 3 | H | H | |
| 36 | N | S | 3 | H | H | |
| 37 | CH | S | 3 | 4-Cl | H | |
| 38 | N | S | 3 | 4-Cl | H | |

Verbindungen der Formel II für die W die zur Vervollständigung eines Naphthalin, Tetrahydronaphthalin- bzw. Thiophen-Ringsystem benötigten C-Atome bedeutet.

Nr.

39 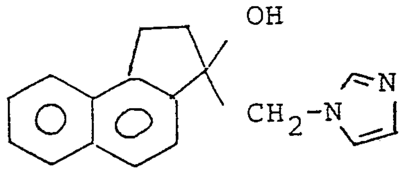 Fp. 177-178°C

40 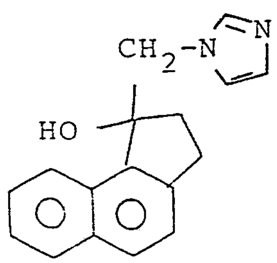 Fp. 184-186°C

41 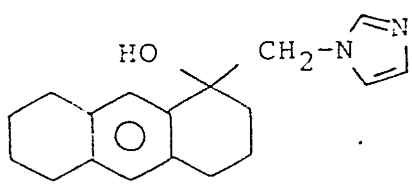 Fp. 153-155°C

Nr.

42 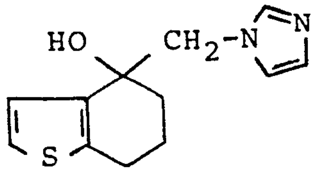 Fp. 205-210°C

43 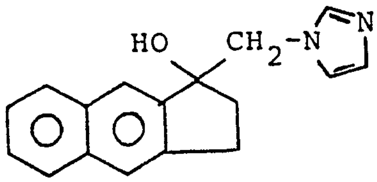

44 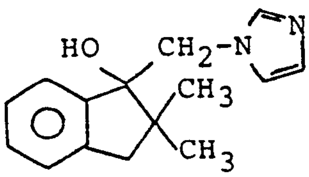 Fp. 142-144°C

45 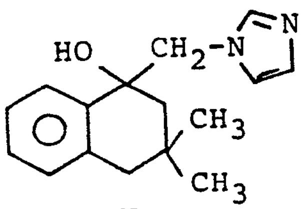 Fp. 140-142°C

46 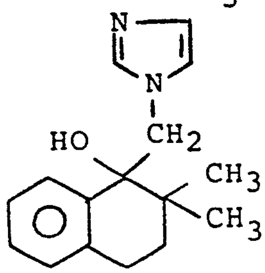 Fp. 163-165°C

47 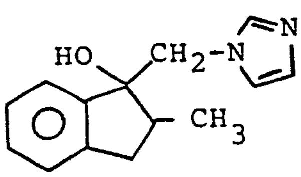

48 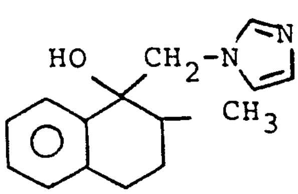

Nr.

49

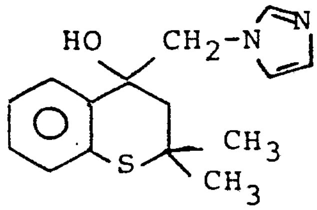

50

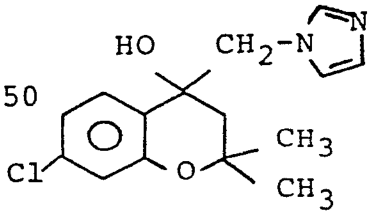

51

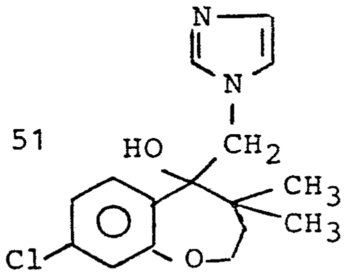

Biologische Beispiele

A) Arzneimittel

Die in vitro-Prüfungen auf antimykotische Wirkung wurden im Reihenverdünnungstest an Hauptpilzen (Trichophyton mentagrophytes, Trichophyton rubrum, Microsporum canis), Hefepilzen (Candida Albicans) und Schimmelpilzen (Aspergillus niger) durchgeführt (Material und Methodik: Mikrotitrationstechnik veröffentlicht in Mykosen 27, 14 (1984). Wie aus Tabelle 4 ersichtlich, zeigen bei diesen in vitro-Prüfungen die erfindungsgemäßen Verbindungen gute antimykotische Eigenschaften, die denjenigen der Referenzsubstand Clotrimazol überlegen sind.

Tabelle 4

Minimale Hemmkonzentration µg/ml

| Präparat Verbindung Nr. ᵃlle 1 | Trichophyton mentagrophytes (100/25) | Trichophyton rubrum (101/58) | Microsporum canis (150/120) | Candida albicans (200/175) | Aspergillus niger (500/284) |
|---|---|---|---|---|---|
| 20 | 0.97 | 0.97 | 0.97 | 1.95 | 0.97 |
| 36 | 0.12 | 0.12 | 0.12 | 1.95 | 0.97 |
| 60 | 0.24 | 0.06 | 0.007 | 0.48 | 1.95 |
| 13 | 0.48 | 0.06 | 0.06 | 0.97 | 1.95 |
| 70 | 0.48 | 0.12 | 0.06 | 0.97 | 0.97 |
| 67 | 0.97 | 0.97 | 0.06 | 1.95 | 1.95 |
| Clotrimazol | 0.97 | 0.97 | 0.97 | 3.9 | 1.95 |

Material und Methodik: Mikrotitrationstechnik veröffentlicht in Mykosen 27, 14 Heft 1 (1984)

Als Beispiel für die hohe lokale in vivo Wirksamkeit der erfindungsgemäßen Verbindungen werden Behandlungsergebnisse an experimentell mit Trichophyton mentagrophytes infizierten Labortieren angeführt.

Zur Feststellung der lokalen Wirksamkeit wurden jeweils zwei 450-500 g schwere Meerschweinchen (Stamm Pirbright white) mit 1,5 x $10^4$ Konidien/Tier in die Epidermis, verteilt auf 6 Infektionspunkte, infiziert.

Die Behandlung der Tiere erfolgte dermal ab dem 3. Tag nach der Infektion an 5 aufeinanderfolgenden Tagen durch Aufbringen einer 0,1 %igen Präparatlösung auf 3 Infektionsstellen einer Rückenseite. Die andere Rückenseite wurde mit Vehikel ohne Präparat auf gleiche Weise behandelt.

Neben den mit den erfindungsgemäßen Substanzen behandelten Tieren wurden zwei Tiere mit der Referenzsubstanz Clotrimazol behandelt, zwei Tiere blieben nach der Infektion unbehandelt.

Wie in Tabelle 5 ersichtlich, zeigten die erfindungsgemäßen Verbindungen eine deutlich stärkere Reduzierung der Mykosendurchmesser als das Standardpräparat Clotrimazol d.h. der antimykotische Effekt der erfindungsgemäßen Verbindung war demjenigen von Clotrimazol bis zu 80 % überlegen.

Tabelle 5

| Konzentration | Präparat Verbindung Nr. Tabelle 1 | Mykosen (Durchmesser in mm) Vehikelkontrollen $x_1$ | (s) | $n^{1)}$ | Präparat + Vehikel $x_2$ | (s) | $n^{1)}$ | Differenz $x_1 - x_2$ | (%) |
|---|---|---|---|---|---|---|---|---|---|
| Dermal | | | | | | | | | |
| 5 x 0.1 % | 20 | 14 | (1.7) | 6 | 4.3 | (1.8) | 6 | 9.7 | (138.5) |
| | 36 | 14.6 | (3) | 6 | 3.3 | (1.5) | 6 | 11.3 | (161.4) |
| | 60 | 15.5 | (2.3) | 6 | 4.5 | (0.8) | 6 | 11 | (157.1) |
| | 13 | 14.5 | (2.5) | 6 | 3.7 | (0.9) | 6 | 10.8 | (154.2) |
| | 70 | 15.8 | (3.2) | 6 | 3 | (0.8) | 6 | 12.8 | (182.8) |
| | 67 | 10.7 | (1.2) | 6 | 3.2 | (0.8) | 6 | 7.5 | (107.1) |
| | Clotrimazol | 14.6 | (1.7) | 6 | 7.6 | (0.4) | 6 | 7 | (100) |
| Kontrollen infizierte Tiere unbehandelt | - | 14 | (2.2) | 12 | | - | | - | |

n = Anzahl Meßwerte

Die antikonvulsive Wirkung der Verbindungen I und II wird durch die Daten von Tabelle 10 belegt, welche wie folgt bestimmt wurden:

Antagonismus gegen Pentetrazol-induzierte Krämpfe (Maus) (PTZ)

Methode:

Gruppen von 10 männlichen Mäusen (Stamm: NMRI, SPF-71, KF) im Gewicht von 18-22 g werden in diesem Versuch verwendet. Die zu prüfenden Substanzen werden in einer 1 %igen Aufschwemmung von Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz.

Pentetrazol (Cardiazol (R)) wird in wäßriger Lösung in einer Dosis von 125 mg/kg Körpergewicht 1 Stunde nach Verabreichung der Prüfsubstanzen subcutan injiziert. Diese Dosis erzeugt bei 90-100 % der Kontrolltiere innerhalb der Beobachtungszeit von 60 Minuten nach der Injektion tonische Extensorkrämpfe der hinteren Extremitäten. Als Schutzwirkung der Prüfsubstanz wird gewertet, wenn die Anzahl der krampfenden Tiere gegenüber der Kontrollgruppe vermindert ist.

Bei vorhandener Schutzwirkung der Prüfsubstanz wird eine mittlere effektive Dosis (ED-50) grafisch (Litchfield and Wilcoxon) oder rechnerisch (Probitanalyse) ermittelt.

Literatur: E.A. SWINYARD, J.Pharmacol.Exp. Ther. 106, (1952), 319-330

Antagonismus gegen Elektroschock-induzierte Krämpfe (Maus) (ECS)

Methode:

Gruppen von 6 männlichen Mäusen (Stamm: NMRI, SPF-71, KF) im Gewicht von 18-22 g werden in diesem Versuch verwendet.

Die zu prüfenden Substanzen werden in einer 1 %igen Aufschwemmung von Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz.
Eine Stunde nach Prüfsubstanzgabe wird den Tieren über Cornea-Elektroden ein Elektroschock verabreicht (Stromstärke 12-22 mA, Dauer 0.2 sec. 50 Hz), nachdem vorher an einer zweiten Kontrollgruppe die Stromstärke ermittelt worden war, die bei 100 % der Tiere einen tonischen Extensorkrampf der hinteren Extremitäten hervorruft.
Die Anzahl der durch die Prüfsubstanz vor einem Extensorkrampf geschützten Tiere dient als Maß der antikonvulsiven Wirkung in diesem Test.
Bei vorhandener Schutzwirkung der Prüfsubstanz wird eine mittlere effektive Dosis (ED-50) grafisch (Litchfield and Wilcoxon) oder rechnerisch (Probitanalyse) ermittelt.

Literatur: E.A. SWINYARD, In: Experimental Models of Epilepsy, Raven Press, New York, 1972, p. 433-458.

Tabelle 10

| Nr. | $ED_{50}$ (mg/kg p.o.) Ptz | ECS | $LD_{50}$ (mg/kg per os) | |
|---|---|---|---|---|
| Verb. 59, Tab. 1 | 46 | 34 | | |
| Verb. 13, Tab. 1 | 16 | 17.3 | >300 | |
| Verb. 53, Tab. 1 | 30 | 35.1 | >300 | |
| Verb. 33, Tab. 1 | 55 | 24.6 | | |
| Verb. 65, Tab. 1 | 15.4 | 55.1 | | |
| Verb. 32, Tab. 1 | 17.2 | 60 | | |
| Verb. 1 , Tab. 3 | 70 | 18.2 | 300 | |
| Verb. 11, Tab. 3 | 9,3 | | | |

## Biologische Beispiele

B.) Pflanzenschutz

### Versuch I

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation werden die Pflanzen mit den in Tabelle 6 aufgeführten Verbindungen in den angegebenen Wirkstoffkonzentrationen gleichmäßig benetzt. Nach einer Inkubationszeit von 10 Tagen werden die Pflanzen auf den Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle 6 zusammengefaßt.

### Tabelle 6

| Verbindung Nr. Tabelle 1 | mit Weizenmehltau befallene Blattfläche in % bei mg Wirkstoff/Liter Spritzbrühe | | | |
|---|---|---|---|---|
| | 500 | 250 | 125 | 60 |
| 21 | 0 | 0 | 0 | 0 |
| 23 | 0 | 0 | 0 | 0 |
| 2 | 0 | 0 | 0 | 0 |
| 52 | 0 | 0 | 0 | 0 |
| 34 | 0 | 0 | 0 | 0 |
| 33 | 0 | 0 | 0 | 0 |
| 29 | 0 | 0 | 0-3 | 3 |
| 123 | 0 | 0 | 0-3 | 3 |
| 51 | 0 | 0 | 0 | 0-3 |
| 20 | 0 | 0 | 0-3 | 3 |
| 31 | 0 | 0 | 0 | 0 |
| 64 | 0 | 0 | 3 | 3-5 |

Fortsetzung Tabelle 6

| Verbindung Nr. | mit Weizenmehltau befallene Blattfläche in % bei mg Wirkstoff/Liter Spritzbrühe | | | |
|---|---|---|---|---|
| | 500 | 250 | 125 | 60 |
| 34 (Tab. 1) | 0 | 0 | 0-3 | 3 |
| 52 " | 0 | 0 | 0 | 0 |
| 2 " | 0 | 0 | 0-3 | 0-3 |
| 23 " | 0 | 0 | 0 | 0-3 |
| 21 | 0 | 0 | 0 | 0 |
| Bsp. Nr. 36 | 0 | 0 | 0-3 | 0-3 |
| 42 (Tab. 3) | 0 | 0 | 0-3 | 3 |
| 10 " | 0 | 0 | 0 | 0-3 |
| unbehandelte, infizierte Pflanzen | | | 100 | |

Versuch II

Gurkenpflanzen (Sorte Delikateß) werden im 2-Blattstadium mit einer Konidiensuspension von Gurkenmehltau (Erysiphe cichoracearum) stark inokuliert. Nach einer Abtrocknungszeit der Sporensuspension von 30 Minuten werden die Pflanzen in einem Gewächshaus bei 22°C und 90 % relativer Luftfeuchte aufgestellt.

3 Tage nach Infektion werden die Pflanzen mit den in Tabelle 7 genannten Verbindungen und Wirkstoffkonzentrationen troßfnaß gleichmäßig benetzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in Tabelle 7 zusammengefaßt.

Tabelle 7

| Verbindung Nr. | mit Gurkenmehltau befallene Blattfläche in % bei mg Wirkstoff/Liter Spritzbrühe | | | |
|---|---|---|---|---|
| | 500 | 250 | 125 | 60 |
| 21 (Tab. 1) | 0 | 0 | 0 | 0 |
| 23 " | 0 | 0 | 0 | 0 |
| 52 " | 0 | 0 | 0 | 0 |
| 31 | 0 | 0 | 0 | 0 |
| 10 (Tab. 3) | 0 | 0 | 0 | 0 |
| unbehandelte, infizierte Pflanzen | | | 100 | |

Versuch III

Apfelunterlagen (BM IX) wurden im 4-Blattstadium mit den beanspruchten Verbindungen in den in Tabelle 8 genannten Anwendungskonzentrationen gleichmäßig benetzt. Nach Antrocknen des Wirkstoffbelages wurden die Pflanzen mit Konidien des Apfelschorfs (Venturia inaequalis) stark infiziert und tropfnaß in eine Klimakammer gestellt, deren Temperatur 22°C und deren relative Luftfeuchtigkeit 100 % betrug. Nach einer Infektionszeit von 48 Stunden kamen die Pflanzen in ein Gewächshaus mit 18°C und einer relativen Luftfeuchte von 95 - 100 %.

Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad der Pflanzen mit Apfelschorf wurde in % befallener Blattfläche, bezogen auf unbehandelte, infizierte Pflanzen, ausgedrückt und ist in Tabelle 8 wiedergegeben.

Tabelle 8

Verbindung Nr. % Schorfbefall bei mg Wirkstoff/l Spritzbrühe

| | 500 | 250 | 125 | 60 |
|---|---|---|---|---|
| 21 (Tab. 1) | 0 | 0 | 0 | 0-3 |
| 2 " | 0 | 0 | 0 | 0 |
| 52 " | 0 | 0 | 0 | 0-3 |
| 59 " | 0 | 0 | 0 | 0-3 |
| 34 " | 0 | 0 | 0 | 0-3 |
| 33 " | 0 | 0 | 0 | 0-3 |
| 42 (Tab. 3) | 0 | 0 | 0 | 0-3 |
| unbehandelte, infizierte Pflanzen | | | 100 | |

Versuch IV

Zuckerrübenpflanzen wurden im 6-Blattstadium mit den beanspruchten Verbindungen in den in Tabelle 9 genannten Aufwandmengen gleichmäßig benetzt. Nach Antrocknen des Wirkstoffbelages wurden die Pflanzen mit Konidien des Erregers der Blattfleckenkrankheit der Rübe (Cercospora beticola) stark inokuliert und tropfnaß in eine Klimakammer mit ca. 100 % relativer Luftfeuchte und 25°C gestellt. 48 Stunden später kamen die Pflanzen in ein Gewächshaus zurück. 14 Tage später wurden sie auf Befall mit der Blattfleckenkrankheit untersucht. Der Befallsgrad wurde in % befallener Blattfläche, bezogen auf unbehandelte, infizierte Kontrollpflanzen (= 100 %), ausgedrückt. Die Ergebnisse sind in Tabelle 9 zusammengestellt.

Tabelle 9

| Verbindung Nr. Tabelle 1 | mit Cercospora beticola befallene Blattfläche in % bei mg Wirkstoff pro Liter Spritzbrühe | | | |
|---|---|---|---|---|
| | 500 | 250 | 125 | 60 |
| 23 | 0 | 0 | 0 | 0-3 |
| 2 | 0 | 0 | 0 | 0 |
| 59 | 0 | 0 | 0 | 0-5 |
| 34 | 0 | 0 | 0 | 0-3 |
| 33 | 0 | 0 | 0 | 0 |
| unbehandelte, infizierte Pflanzen | | | 100 | |

Versuch V

Biomalzagar wurde mit den beanspruchten Verbindungen in den in Tabelle 10 angegebenen Konzentrationen versetzt. Nach dem Erstarren des Agars erfolgte die Beimpfung mit Pseudocercosporella-Kulturen. Jeweils 20 µl einer Sporensuspension wurden auf das Zentrum der Agarplatte aufgebracht. Die Versuche wurden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle 10

| Verbindung | Wirkungsgrad gegenüber Pseudocercosporella herpotrichoides in % bei ppm Wirkstoff | | |
|---|---|---|---|
| Nr. | 500 | 250 | 125 |
| 75 (Tab. 1) | 100 | 100 | 100 |
| 64 " | 100 | 100 | 100 |
| 66 " | 100 | 100 | 100 |
| 73 " | 100 | 100 | 100 |
| 71 " | 100 | 100 | 100 |
| 80 " | 100 | 100 | 100 |
| 64 " | 100 | 100 | 100 |
| 33 " | 100 | 100 | 100 |
| 59 " | 100 | 100 | 100 |
| 25 " | 100 | 100 | 100 |
| 32 " | 100 | 100 | 100 |
| 50 " | 100 | 100 | 100 |
| 79 " | 100 | 100 | 100 |
| 40 " | 100 | 100 | 100 |
| 60 " | 100 | 100 | 100 |
| 53 " | 100 | 100 | 100 |
| 36 " | 100 | 100 | 100 |
| 30 " | 100 | 100 | 100 |
| 24 " | 100 | 100 | 100 |
| 13 " | 100 | 100 | 100 |
| 86 " | 100 | 100 | 100 |
| 10 (Tab. 3) | 100 | 100 | 100 |
| 11 " | 100 | 100 | 100 |
| Kontrolle | | 100 | |

PATENTANSPRÜCHE:

0236913

1. Azolverbindungen der Formel (I)

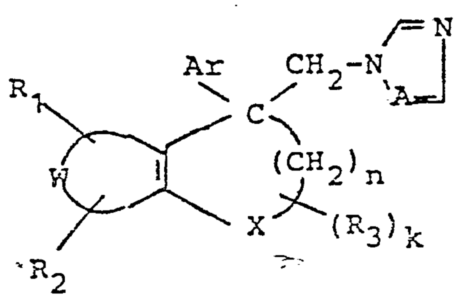

(I)

worin

A CH oder N bedeutet

k 1 oder 2 bedeutet

n 1, 2 oder 3 bedeutet

Ar Phenyl, Biphenyl, Phenoxyphenyl, Phenoxythienyl, Phenylthiophenyl, Phenylsulfinylphenyl, Phenylsulfonylphenyl, Benzylphenyl, Benzyloxyphenyl, Benzylthiophenyl, Fluorenyl, Indanyl, 1,2,3,4-Tetrahydronaphthyl, Naphthyl oder Thienyl bedeutet, wobei die Benzolringe, das Naphthalinsystem oder der Thiophenring unsubstituiert oder mit einem oder zwei Subsituenten substituiert sein können, die gleich oder verschieden sind und jeweils Fluor, Chlor, Brom, Jod, $(C_1-C_8)$-Alkyl, verzweigt oder unverzweigt, unsubstituiert oder durch 1-9 Fluor- und/oder Chloratom substituiert, $(C_3-C_8)$-Cycloalkyl, $(C_1-C_4)$-Alkoxy, $(C_1-C_4)$-Alkylthio, Hydroxy, Nitro oder Cyano bedeuten,

W die zur Vervollständigung eines Benzol-, Naphthalin- oder Thiophen-Ringsystems nötigen Atome bedeutet,

X ein Rest $>C(R_3)_2$, sowie für den Fall, daß W die zur Vervollständigung eines Benzol- oder Naphthalin-Ringsystems nötigen Atome bedeutet, auch Sauerstoff oder Schwefel bedeutet,

$R^1$ und $R^2$ gleich oder verschieden sind und jeweils Wasserstoff, Fluor, Chlor, Brom, Jod, $(C_1-C_8)$-Alkyl, unverzweigt oder verzweigt, unsubstituiert oder mit 1-9 Fluor- oder Chloratomen substituiert, $(C_3-C_8)$Cycloalkyl, $(C_1-C_8)$-Alkoxy, $(C_1-C_8)$-Alkylthio, Cyano oder Nitro bedeuten,

sowie für den Fall, daß gleichzeitig W die zur Vervollständigung eines Benzolrings nötigen Atome bedeutet,

$R^1$ auch Phenyl, Phenoxy, Phenylthio, Phenylsulfinyl, Phenylsulfonyl, Benzyl, Benzyloxy oder Hydroxy bedeutet, wobei die Phenylgruppen in diesen Substituenten unsubstituiert oder durch einen oder zwei Substituenten substituiert sein können und diese Substituenten gleich oder verschieden sind und jeweils Fluor, Chlor, Brom, Jod, $(C_1-C_4)$-Alkyl, verzweigt oder unverzweigt, unsubstituiert oder durch 1-9 Fluor- oder Chloratome substituiert, $(C_3-C_8)$-Cycloalkyl, $(C_1-C_4)$-Alkoxy, $(C_1-C_4)$-Alkylthio, Cyano oder Nitro bedeuten, oder $R^1$, $R^2$, sofern sie orthoständig orientiert sind, zusammen eine $(C_3-C_5)$-Alkylenkette bedeuten und $R^3$ als Substituent der Kette $(CH_2)_n$ oder von X unabhängig voneinander H oder $(C_1-C_8)$-Alkyl bedeutet oder zwei Reste $R^3$, wenn sie an dasselbe C-Atom gebunden sind eine $(C_2-C_6)$-Alkylenkette bedeuten sowie ihre Stereoisomeren und ihre Salze.

2. Verbindung nach Anspruch 1, dadurch gekennzeichnet, daß mindestens einer der Substituenten oder Indices folgende Bedeutung hat:
   - W die zur Vervollständigung eines Benzol-Ringsystems nötigen Atome bedeutet,
   - X die Methylengruppe, Sauerstoff oder Schwefel bedeutet,
   - A CH oder N bedeutet,
   - n 1, 2 oder 3 bedeutet,
   - Ar Phenyl, 4-Biphenylyl, 4-Phenoxyphenyl, 4-Phenylthiophenyl, Fluorenyl, Indanyl, 1,2,3,4-Tetrahydronaphthyl oder Thienyl bedeutet; unsubstituiert oder an den Benzolringen oder a. Thiophenring mit einem

oder zwei Substituenten substituiert, die gleich oder verschieden sein können und jeweils Fluor, Chlor, Brom, $(C_1-C_4)$-Alkyl, $(C_3-C_8)$-Cycloalkyl, $(C_1-C_4)$-Alkoxy oder Trifluormethyl bedeuten,

$R^1$ und $R^2$ gleich oder verschieden sind und jeweils Wasserstoff, Fluor, Chlor, Brom, Trifluormethyl, $(C_1-C_8)$-Alkyl oder $(C_1-C_8)$Alkoxy bedeuten.

3. Verbindung nach Anspruch 2, dadurch gekennzeichnet, daß Ar Mono-Halogenphenyl oder 2,4-Dihalogenphenyl bedeutet, wobei die Halogenatome gleich oder verschieden sind und jeweils Fluor oder Chlor bedeuten oder Ar 4-Biphenylyl, 4-Phenoxyphenyl, 4-Phenylthiophenyl oder Fluorenyl bedeutet, unsubstituiert oder an einem Benzolring mit einem oder zwei Substituenten substituiert und diese Substituenten gleich oder verschieden sind und jeweils Fluor, Chlor, Trifluormethyl, Methyl oder Methoxy bedeuten.

4. Verbindung nach Anspruch 3, dadurch gekennzeichnet, daß mindestens einer der Substituenten und Indices folgende Bedeutung hat:
Ar 4-Chlorphenyl, 4-Fluorphenyl, 2,4-Dichlorphenyl, 2,4-Difluorphenyl, 4-Chlor-2-fluorphenyl, Fluorenyl, 4-Biphenylyl, 4-Phenoxyphenyl oder 4-Phenylthiophenyl bedeutet, wobei die drei letztgenannten Gruppen unsubstituiert oder in der 4'-Position mit Fluor, Chlor, Methyl, Methoxy oder der Trifluormethylgruppe substituiert sein können, $R^1$ und $R^2$ gleich oder verschieden sind und Wasserstoff, Fluor, Chlor, Trifluormethyl, Methyl oder Methoxy bedeuten.

5. Verfahren zur Herstellung der Verbindungen der Formel (I) nach Ansprüchen 1,2,3 oder 4, dadurch gekennzeichnet, daß man

a) eine Verbindung der Formel (II)

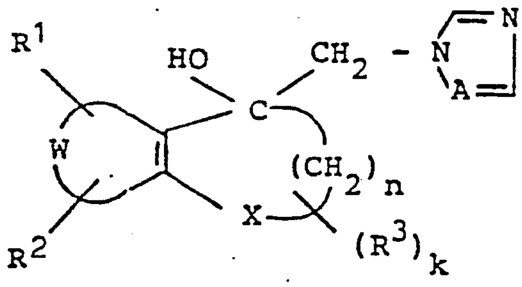

(II)

mit einer Verbindung der Formel (III)

ArH (III)

worin Ar die in Anspruch 1 angegebenen Bedeutungen hat, in Gegenwart einer Lewis-Säure oder einer starken Protonensäure unter Wasserabspaltung zu einer Verbindung der Formel (I) umsetzt, oder

b) eine Verbindung der Formel (IV),

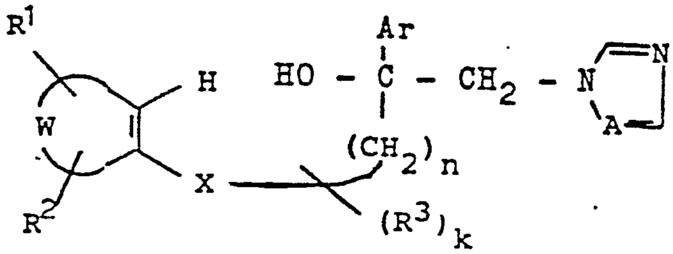

(IV)

in Gegenwart einer Lewis-Säure oder einer starken Protonen-Säure unter Wasserabspaltung zu einer Verbindung der Formel (I) umsetzt oder

c) eine der nach a) oder b) erhaltenen Verbindungen der Formel (I), für die Ar eine mit einer Alkoxygruppe substituierte Phenyl- oder Naphthylgruppe bedeutet oder für die $R^1$ eine Alkoxygruppe bedeutet, einer Etherspaltung unterwirft und die so erhaltenen Verbindungen der Formel (I), für die $R^1$ die Hydroxygruppe bedeutet oder für die

Ar eine mit einer Hydroxygruppe substituierte Phenyl- oder Naphthylgruppe bedeutet, mit einer Verbindung der Formel (V),

$$E - R^4 \quad (V)$$

worin E eine reaktive Abgangsgruppe bedeutet und

$R^4$ eine ($C_1$-$C_8$)-Alkylgruppe oder Benzylgruppe bedeutet, umsetzt, oder

d) eine Verbindung der Formel (I) an einer Phenylthiogruppe zur Phenylsulfinyl- oder Phenylsulfonylgruppe oxidiert.

und eine nach a), b), c) oder d) erhaltene Verbindung der Formel (I) in Form der freien Base oder eines Säureadditionssalzes isoliert.

6. Verbindungen der Formel (II)

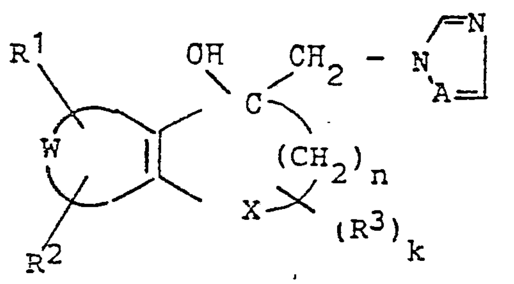

(II)

mit Einschränkung, daß (II) nicht die Verbindungen 1-Hydroxy-1-(azol-1-ylmethyl)-1,2,3,4-tetrahydronaphthalin bedeutet, wobei unter "Azol" 1H-Imidazol oder 1,2,4-Triazol zu verstehen ist.

7. Verfahren zur Herstellung von Verbindungen der Formel (II) nach Anspruch 6, dadurch gekennzeichnet, daß man eine Verbindung der Formel (VI)

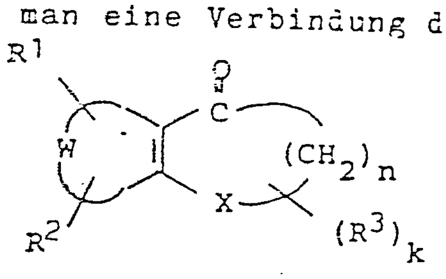

(VI)

mit einem Schwefelylid der Formel (VII)

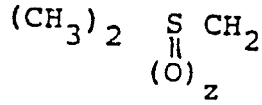

(VII)

worin z O oder 1 bedeutet,
zu einer Verbindung der Formel (VIII) umsetzt,

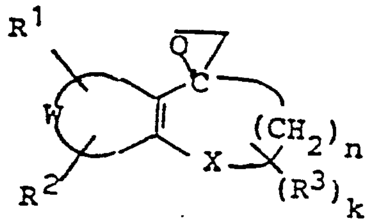

(VIII)

und anschließend die Verbindung der Formel (VIII) mit einer Verbindung der Formel (IX)

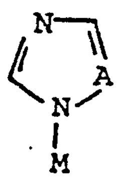

(IX)

worin A die in Anspruch 1 angegebenen Bedeutungen hat und M Wasserstoff, die Trimethylsilylgruppe oder ein Metalläquivalent bedeutet, zu einer Verbindung der Formel (II) umsetzt.

8. Verbindungen der Formel (IV),

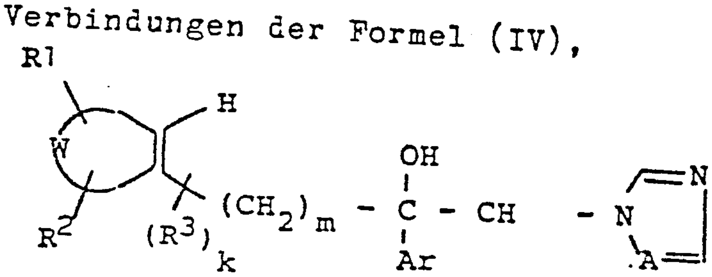

(IV)

worin A, Ar, W, $R^1$, $R^2$, $R^3$ und k die in Anspruch 1 angegebenen Bedeutungen haben und m 3 oder 4 bedeutet.

9. Verfahren zur Herstellung von Verbindungen der Formel (IV) nach Anspruch 8, dadurch gekennzeichnet, daß man eine Verbindung der Formel (X)

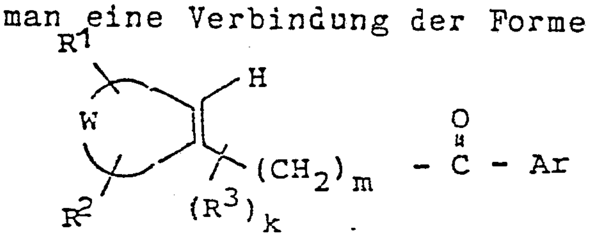 (X)

mit einem Schwefelylid der Formel (VII)

$(CH_3)_2\underset{(O)_z}{S}CH_2$ (VII)

worin z 0 oder 1 bedeutet,
zu einer Verbindung der Formel (XI) umsetzt

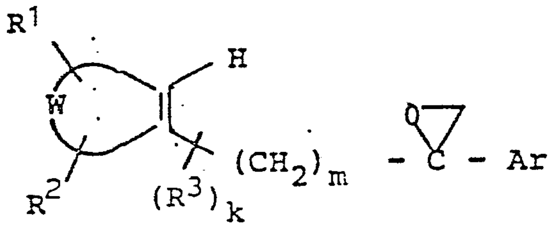 (XI)

und die Verbindung der Formel (XI) anschließend mit einer Verbindung der Formel (IX) nach Anspruch 7 zu einer Verbindung der Formel (IV) umsetzt.

10. Verfahren zur Herstellung von Verbindungen der Formel (X)

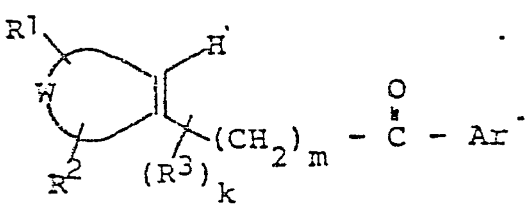 (X)

worin W, Ar, $R^1$, $R^2$, $R^3$ und k die in Anspruch 1 angegebenen Bedeutungen haben und m = 3 ist, dadurch gekennzeichnet, daß man eine Verbindung der Formel (XII)

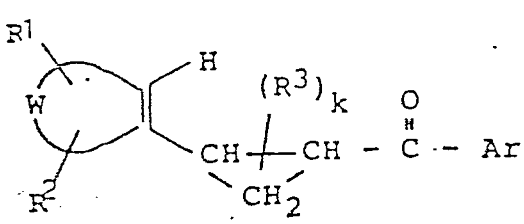 (XII)

in Gegenwart eines Metallkatalysators hydriert und so eine Verbindung der Formel (X) erhält.

11. Arzneimittel, dadurch gekennzeichnet, daß es mindestens eine Verbindung (I) gemäß Anspruch 1, 2, 3 oder 4 enthält oder aus ihr besteht.

12. Verwendung einer Verbindung I nach Anspruch 1, 2, 3 oder 4 zur Herstellung eines Medikaments zur Bekämpfung von Mykosen, Protozoen und grampositiver und gramnegativer Bakterien.

13. Verwendung einer Verbindung (I) nach Anspruch 1, 2, 3 oder 4 zur Herstellung eines Medikaments zur Behandlung und Verhütung von Konvulsionen.

14. Schädlingsbekämpfungsmittel, gekennzeichnet durch einen Gehalt an einer Verbindung der Formel (I) nach Anspruch 1.

15. Verfahren zur Bekämpfung oder Verhinderung eines Befalls von Kulturpflanzen durch phytopathogene Pilze, dadurch gekennzeichnet, daß man eine Verbindung der Formel (I) gemäß Anspruch 1, 2, 3 oder 4 auf die Pflanze bzw. auf durch Pilzbefall bedrohte Flächen, Pflanzen oder Saatgüter einwirken läßt.

16. Verwendung einer Verbindung II nach Anspruch 6 zur Herstellung eines Medikaments zur Behandlung und Verhütung von Konvulsionen.

17. Verwendung einer Verbindung der Formel II nach Anspruch 6 als Fungizid.

Patentansprüche Griechenland und Österreich:

1. Verfahren zur Herstellung von Azolverbindungen der Formel (I)

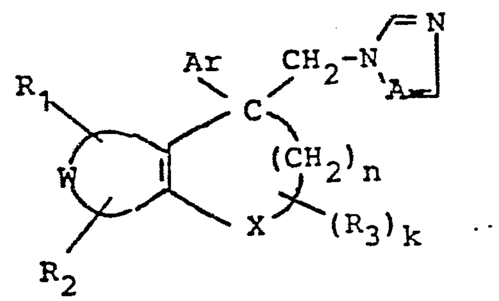

(I)

worin

A CH oder N bedeutet

k 1 oder 2 bedeutet

n 1, 2 oder 3 bedeutet

Ar Phenyl, Biphenyl, Phenoxyphenyl, Phenoxythienyl, Phenylthiophenyl, Phenylsulfinylphenyl, Phenylsulfonylphenyl, Benzylphenyl, Benzyloxyphenyl, Benzylthiophenyl, Fluorenyl, Indanyl, 1,2,3,4-Tetrahydronaphthyl, Naphthyl oder Thienyl bedeutet, wobei die Benzolringe, das Naphthalinsystem oder der Thiophenring unsubstituiert oder mit einem oder zwei Subsituenten substituiert sein können, die gleich oder verschieden sind und jeweils Fluor, Chlor, Brom, Jod, $(C_1-C_8)$-Alkyl, verzweigt oder unverzweigt, unsubstituiert oder durch 1-9 Fluor- und/oder Chloratom substituiert, $(C_3-C_8)$-Cycloalkyl, $(C_1-C_4)$-Alkoxy, $(C_1-C_4)$-Alkylthio, Hydroxy, Nitro oder Cyano bedeuten,

W die zur Vervollständigung eines Benzol-, Naphthalin- oder Thiophen-Ringsystems nötigen Atome bedeutet,

X ein Rest $>C(R_3)_2$, sowie für den Fall, daß W die zur Vervollständigung eines Benzol- oder Naphthalin-Ringsystems nötigen Atome bedeutet, auch Sauerstoff oder Schwefel bedeutet,

$R^1$ und $R^2$ gleich oder verschieden sind und jeweils Wasserstoff, Fluor, Chlor, Brom, Jod, $(C_1-C_8)$-Alkyl, unverzweigt oder verzweigt, unsubstituiert oder mit 1-9 Fluor- oder Chloratomen substituiert, $(C_3-C_8)$Cycloalkyl, $(C_1-C_8)$-Alkoxy, $(C_1-C_8)$-Alkylthio, Cyano oder Nitro bedeuten,

sowie für den Fall, daß gleichzeitig W die zur Vervollständigung eines Benzolrings nötigen Atome bedeutet,

$R^1$ auch Phenyl, Phenoxy, Phenylthio, Phenylsulfinyl, Phenylsulfonyl, Benzyl, Benzyloxy oder Hydroxy bedeutet, wobei die Phenylgruppen in diesen Substituenten unsubstituiert oder durch einen oder zwei Substituenten substituiert sein können und diese Substituenten gleich oder verschieden sind und jeweils Fluor, Chlor, Brom, Jod, $(C_1-C_4)$-Alkyl, verzweigt oder unverzweigt, unsubstituiert oder durch 1-9 Fluor- oder Chloratome substituiert, $(C_3-C_8)$-Cycloalkyl, $(C_1-C_4)$-Alkoxy, $(C_1-C_4)$-Alkylthio, Cyano oder Nitro bedeuten, oder $R^1$, $R^2$, sofern sie orthoständig orientiert sind, zusammen eine $(C_3-C_5)$-Alkylenkette bedeuten und $R^3$ als Substituent der Kette $(CH_2)_n$ oder von X unabhängig voneinander H oder $(C_1-C_8)$-Alkyl bedeutet oder zwei Reste $R^3$, wenn sie an dasselbe C-Atom gebunden sind, eine $(C_2-C_6)$-Alkylenkette bedeuten sowie ihre Stereoisomeren und ihre Salze, dadurch gekennzeichnet, daß man

a) eine Verbindung der Formel (II)

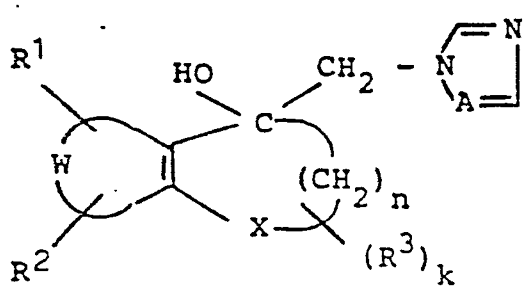

(II)

mit einer Verbindung der Formel (III)

Ar H (III)

worin Ar die in Anspruch 1 angegebenen Bedeutungen hat, in Gegenwart einer Lewis-Säure oder einer

starken Protonensäure unter Wasserabspaltung zu einer Verbindung der Formel (I) umsetzt, oder

b) eine Verbindung der Formel (IV),

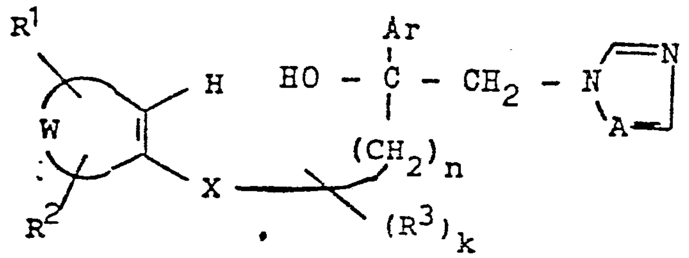

(IV)

in Gegenwart einer Lewis-Säure oder einer starken Protonen-Säure unter Wasserabspaltung zu einer Verbindung der Formel (I) umsetzt oder

c) eine der nach a) oder b) erhaltenen Verbindungen der Formel (I), für die Ar eine mit einer Alkoxygruppe substituierte Phenyl- oder Naphthylgruppe bedeutet oder für die $R^1$ eine Alkoxygruppe bedeutet, einer Etherspaltung unterwirft und die so erhaltenen Verbindungen der Formel (I), für die $R^1$ die Hydroxygruppe bedeutet oder für die Ar eine mit einer Hydroxygruppe substituierte Phenyl- oder Naphthylgruppe bedeutet, mit einer Verbindung der Formel (V),

$$E - R^4 \quad (V)$$

worin E eine reaktive Abgangsgruppe bedeutet und

$R^4$ eine $(C_1-C_8)$-Alkylgruppe oder Benzylgruppe bedeutet, umsetzt, oder

gk, AT

d) eine Verbindung der Formel (I) an einer Phenylthiogruppe zur Phenylsulfinyl- oder Phenylsulfonylgruppe oxidiert.

und eine nach a), b), c) oder d) erhaltene Verbindung der Formel (I) in Form der freien Base oder eines Säureadditionssalzes isoliert.

2. Verfahren nach Anspruch 1, dadurch gekennzeichnet, daß mindestens einer der Substituenten oder Indices der Formel I - IV folgende Bedeutung hat:

W die zur Vervollständigung eines Benzol-Ringsystems nötigen Atome bedeutet,

X die Methylengruppe, Sauerstoff oder Schwefel bedeutet,

A CH oder N bedeutet,

n 1, 2 oder 3 bedeutet,

Ar Phenyl, 4-Biphenylyl, 4-Phenoxyphenyl, 4-Phenylthiophenyl, Fluorenyl, Indanyl, 1,2,3,4-Tetrahydronaphthyl oder Thienyl bedeutet, unsubstituiert oder an den Benzolringen oder am Thiophenring mit einem oder zwei Substituenten substituiert, die gleich oder verschieden sein können und jeweils Fluor, Chlor, Brom, $(C_1-C_4)$-Alkyl, $(C_3-C_8)$-Cycloalkyl, $(C_1-C_4)$-Alkoxy oder Trifluormethyl bedeuten,

$R^1$ und $R^2$ gleich oder verschieden sind und jeweils Wasserstoff, Fluor, Chlor, Brom, Trifluormethyl, $(C_1-C_8)$-Alkyl oder $(C_1-C_8)$Alkoxy bedeuten.

3. Verfahren nach Anspruch 2, dadurch gekennzeichnet, daß Ar Mono-Halogenphenyl oder 2,4-Dihalogenphenyl bedeutet, wobei die Halogenatome gleich oder verschieden sind und jeweils Fluor oder Chlor bedeuten oder Ar 4-Biphenylyl, 4-Phenoxyphenyl, 4-Phenylthiophenyl oder Fluorenyl bedeutet, unsubstituiert oder an einem Benzolring mit einem oder zwei Substituenten substituiert und diese

Substituenten gleich oder verschieden sind und jeweils Fluor, Chlor, Trifluormethyl, Methyl oder Methoxy bedeuten.

4. Verfahren nach Anspruch 3, dadurch gekennzeichnet, daß mindestens einer der Substituenten und Indices folgende Bedeutung hat:
Ar 4-Chlorphenyl, 4-Fluorphenyl, 2,4-Dichlorphenyl, 2,4-Difluorphenyl, 4-Chlor-2-fluorphenyl, Fluorenyl, 4-Biphenylyl, 4-Phenoxyphenyl oder 4-Phenylthiophenyl bedeutet, wobei die drei letztgenannten Gruppen unsubstituiert oder in der 4'-Position mit Fluor, Chlor, Methyl, Methoxy oder der Trifluormethylgruppe substituiert sein können, $R^1$ und $R^2$ gleich oder verschieden sind und Wasserstoff, Fluor, Chlor, Trifluormethyl, Methyl oder Methoxy bedeuten.

5. Verfahren zur Herstellung von Verbindungen der Formel (II)

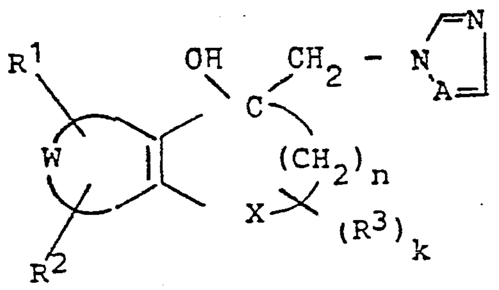

(II)

mit Einschränkung, daß (II) nicht die Verbindungen 1-Hydroxy-1-(azol-1-ylmethyl)-1,2,3,4-tetrahydronaphthalin bedeutet, wobei unter "Azol" 1H-Imidazol oder 1,2,4-Triazol zu verstehen ist, dadurch gekennzeichnet, daß man eine Verbindung der Formel (VI)

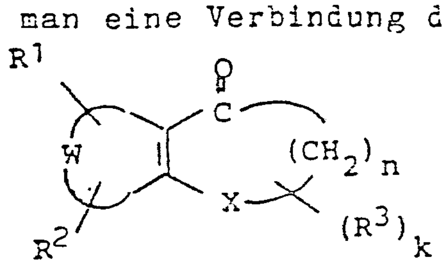

(VI)

mit einem Schwefelylid der Formel (VII)

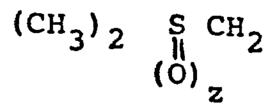

(VII)

worin z 0 oder 1 bedeutet,
zu einer Verbindung der Formel (VIII) umsetzt,

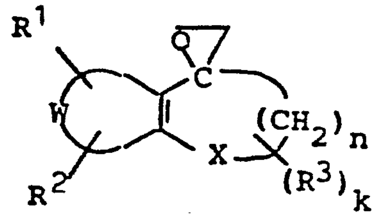

(VIII)

und anschließend die Verbindung der Formel (VIII) mit einer Verbindung der Formel (IX)

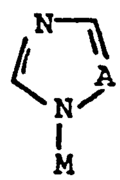

(IX)

worin A die in Anspruch 1 angegebenen Bedeutungen hat und M Wasserstoff, die Trimethylsilylgruppe oder ein Metalläquivalent bedeutet, zu einer Verbindung der Formel (II) umsetzt.

6. Verfahren zur Herstellung von Verbindungen der Formel (IV)

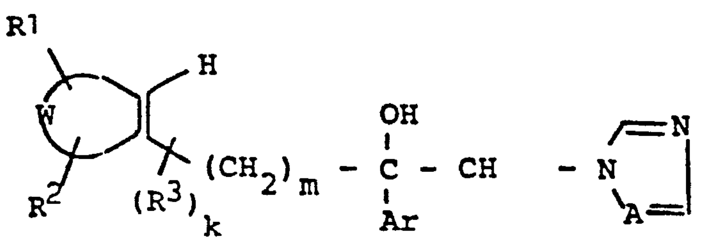

(IV)

worin A, Ar, W, $R^1$, $R^2$, $R^3$ und k die in Anspruch 1 angegebenen Bedeutungen haben und m 3 oder 4 bedeutet, dadurch gekennzeichnet, daß man eine Verbindung der Formel (X)

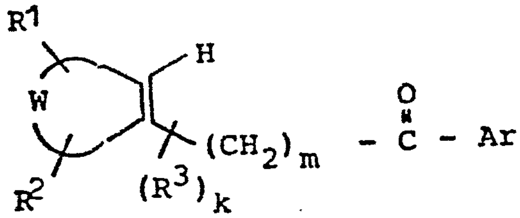

(X)

mit einem Schwefelylid der Formel (VII)

$$(CH_3)_2 \underset{(O)_z}{\overset{}{S}} CH_2 \qquad (VII)$$

worin z 0 oder 1 bedeutet,
zu einer Verbindung der Formel (XI) umsetzt

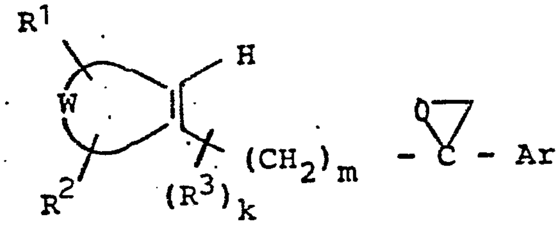

(XI)

und die Verbindung der Formel (XI) anschließend mit einer Verbindung der Formel (IX) nach Anspruch 5 zu einer Verbindung der Formel (IV) umsetzt.

7. Verfahren zur Herstellung von Verbindungen der Formel (X)

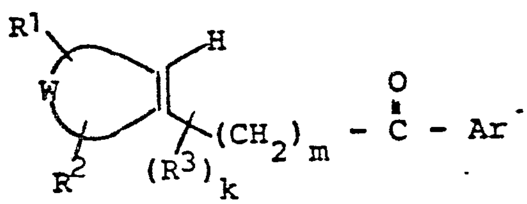

(X)

worin W, Ar, $R^1$, $R^2$, $R^3$ und k die in Anspruch 1 angegebenen Bedeutungen haben und m = 3 ist, dadurch gekennzeichnet, daß man eine Verbindung der Formel (XII)

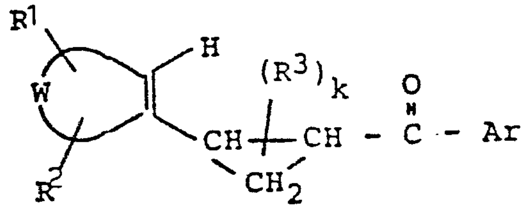

(XII)

in Gegenwart eines Metallkatalysators hydriert und so eine Verbindung der Formel (X) erhält.

8. Verwendung einer Verbindung I nach Ansprüchen 1, 2, 3 oder 4 zur Herstellung eines Medikaments.

9. Verwendung einer Verbindung I nach Ansprüchen 1, 2, 3 oder 4 zur Herstellung eines Medikaments zur Bekämpfung von Mykosen, Protozoen und grampositiver und gramnegativer Bakterien.

10. Verwendung einer Verbindung (I) nach Ansprüchen 1, 2, 3 oder 4 zur Herstellung eines Medikaments zur Behandlung und Verhütung von Konvulsionen.

11. Schädlingsbekämpfungsmittel, gekennzeichnet durch einen Gehalt an einer Verbindung der Formel (I) nach Ansprüchen 1, 2, 3 oder 4.

12. Verfahren zur Bekämpfung oder Verhinderung eines Befalls von Kulturpflanzen durch phytopathogene Pilze, dadurch gekennzeichnet, daß man eine Verbindung der Formel (I) gemäß Ansprüchen 1, 2, 3 oder 4 auf die Pflanze bzw. auf durch Pilzbefall bedrohte Flächen, Pflanzen oder Saatgüter einwirken läßt.

13. Verwendung einer Verbindung II von Anspruch 1 zur Herstellung eines Medikaments zur Behandlung und Verhütung von Konvulsionen.

14. Verwendung einer Verbindung der Formel II von Anspruch 1 als Fungizid.

Patentansprüche Spanien:

1. Verfahren zur Herstellung von Azolverbindungen der Formel (I)

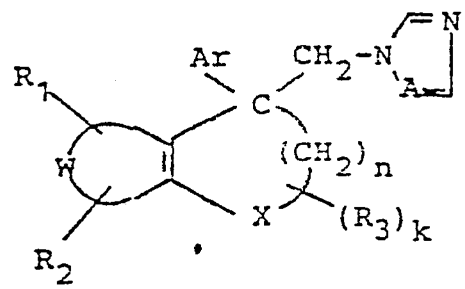

(I)

worin

A CH oder N bedeutet

k 1 oder 2 bedeutet

n 1, 2 oder 3 bedeutet

Ar Phenyl, Biphenyl, Phenoxyphenyl, Phenoxythienyl, Phenylthiophenyl, Phenylsulfinylphenyl, Phenylsulfonylphenyl, Benzylphenyl, Benzyloxyphenyl, Benzylthiophenyl, Fluorenyl, Indanyl, 1,2,3,4-Tetrahydronaphthyl, Naphthyl oder Thienyl bedeutet, wobei die Benzolringe, das Naphthalinsystem oder der Thiophenring unsubstituiert oder mit einem oder zwei Subsituenten substituiert sein können, die gleich oder verschieden sind und jeweils Fluor, Chlor, Brom, Jod, $(C_1-C_8)$-Alkyl, verzweigt oder unverzweigt, unsubstituiert oder durch 1-9 Fluor- und/oder Chloratom substituiert, $(C_3-C_8)$-Cycloalkyl, $(C_1-C_4)$-Alkoxy, $(C_1-C_4)$-Alkylthio, Hydroxy, Nitro oder Cyano bedeuten,

W die zur Vervollständigung eines Benzol-, Naphthalin- oder Thiophen-Ringsystems nötigen Atome bedeutet,

X ein Rest $>C(R_3)_2$, sowie für den Fall, daß W die zur Vervollständigung eines Benzol- oder Naphthalin-Ringsystems nötigen Atome bedeutet, auch Sauerstoff oder Schwefel bedeutet,

$R^1$ und $R^2$ gleich oder verschieden sind und jeweils Wasserstoff, Fluor, Chlor, Brom, Jod, $(C_1-C_8)$-Alkyl, unverzweigt oder verzweigt, unsubstituiert oder mit 1-9 Fluor- oder Chloratomen substituiert, $(C_3-C_8)$Cycloalkyl, $(C_1-C_8)$-Alkoxy, $(C_1-C_8)$-Alkylthio, Cyano oder Nitro bedeuten,

sowie für den Fall, daß gleichzeitig W die zur Vervollständigung eines Benzolrings nötigen Atome bedeutet,

$R^1$ auch Phenyl, Phenoxy, Phenylthio, Phenylsulfinyl, Phenylsulfonyl, Benzyl, Benzyloxy oder Hydroxy bedeutet, wobei die Phenylgruppen in diesen Substituenten unsubstituiert oder durch einen oder zwei Substituenten substituiert sein können und diese Substituenten gleich oder verschieden sind und jeweils Fluor, Chlor, Brom, Jod, $(C_1-C_4)$-Alkyl, verzweigt oder unverzweigt, unsubstituiert oder durch 1-9 Fluor- oder Chloratome substituiert, $(C_3-C_8)$-Cycloalkyl, $(C_1-C_4)$-Alkoxy, $(C_1-C_4)$-Alkylthio, Cyano oder Nitro bedeuten, oder $R^1$, $R^2$, sofern sie orthoständig orientiert sind, zusammen eine $(C_3-C_5)$-Alkylenkette bedeuten und $R^3$ als Substituent der Kette $(CH_2)_n$ oder von X unabhängig voneinander H oder $(C_1-C_8)$-Alkyl bedeutet oder zwei Reste $R^3$, wenn sie an dasselbe C-Atom gebunden sind, eine $(C_2-C_6)$-Alkylenkette bedeuten sowie ihre Stereoisomeren und ihre Salze, dadurch gekennzeichnet, daß man

a) eine Verbindung der Formel (II)

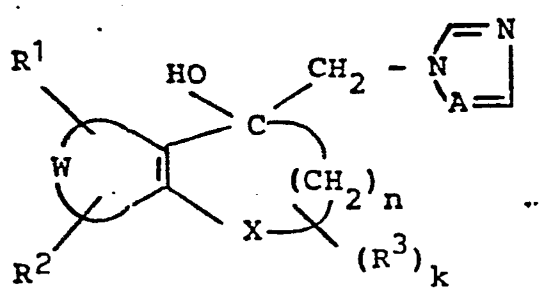

(II)

mit einer Verbindung der Formel (III)

Ar H (III)

worin Ar die in Anspruch 1 angegebenen Bedeutungen hat, in Gegenwart einer Lewis-Säure oder einer

starken Protonensäure unter Wasserabspaltung zu einer Verbindung der Formel (I) umsetzt, oder

b) eine Verbindung der Formel (IV),

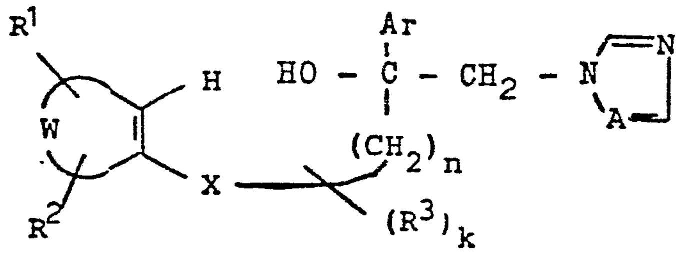

(IV)

in Gegenwart einer Lewis-Säure oder einer starken Protonen-Säure unter Wasserabspaltung zu einer Verbindung der Formel (I) umsetzt oder

c) eine der nach a) oder b) erhaltenen Verbindungen der Formel (I), für die Ar eine mit einer Alkoxygruppe substituierte Phenyl- oder Naphthylgruppe bedeutet oder für die $R^1$ eine Alkoxygruppe bedeutet, einer Etherspaltung unterwirft und die so erhaltenen Verbindungen der Formel (I), für die $R^1$ die Hydroxygruppe bedeutet oder für die Ar eine mit einer Hydroxygruppe substituierte Phenyl- oder Naphthylgruppe bedeutet, mit einer Verbindung der Formel (V),

$$E - R^4 \quad (V)$$

worin E eine reaktive Abgangsgruppe bedeutet und

$R^4$ eine $(C_1-C_8)$-Alkylgruppe oder Benzylgruppe bedeutet, umsetzt, oder

ES

d) eine Verbindung der Formel (I) an einer Phenylthiogruppe zur Phenylsulfinyl- oder Phenylsulfonylgruppe oxidiert.

und eine nach a), b), c) oder d) erhaltene Verbindung der Formel (I) in Form der freien Base oder eines Säureadditionssalzes isoliert.

2. Verfahren nach Anspruch 1, dadurch gekennzeichnet, daß mindestens einer der Substituenten oder Indices der Formel I - IV folgende Bedeutung hat:

W die zur Vervollständigung eines Benzol-Ringsystems nötigen Atome bedeutet,

X die Methylengruppe, Sauerstoff oder Schwefel bedeutet,

A CH oder N bedeutet,

n 1, 2 oder 3 bedeutet,

Ar Phenyl, 4-Biphenylyl, 4-Phenoxyphenyl, 4-Phenylthiophenyl, Fluorenyl, Indanyl, 1,2,3,4-Tetrahydronaphthyl oder Thienyl bedeutet, unsubstituiert oder an den Benzolringen oder am Thiophenring mit einem oder zwei Substituenten substituiert, die gleich oder verschieden sein können und jeweils Fluor, Chlor, Brom, $(C_1-C_4)$-Alkyl, $(C_3-C_8)$-Cycloalkyl, $(C_1-C_4)$-Alkoxy oder Trifluormethyl bedeuten,

$R^1$ und $R^2$ gleich oder verschieden sind und jeweils Wasserstoff, Fluor, Chlor, Brom, Trifluormethyl, $(C_1-C_8)$-Alkyl oder $(C_1-C_8)$Alkoxy bedeuten.

3. Verfahren nach Anspruch 2, dadurch gekennzeichnet, daß Ar Mono-Halogenphenyl oder 2,4-Dihalogenphenyl bedeutet, wobei die Halogenatome gleich oder verschieden sind und jeweils Fluor oder Chlor bedeuten oder Ar 4-Biphenylyl, 4-Phenoxyphenyl, 4-Phenylthiophenyl oder Fluorenyl bedeutet, unsubstituiert oder an einem Benzolring mit einem oder zwei Substituenten substituiert und diese

Substituenten gleich oder verschieden sind und jeweils Fluor, Chlor, Trifluormethyl, Methyl oder Methoxy bedeuten.

4. Verfahren nach Anspruch 3, dadurch gekennzeichnet, daß mindestens einer der Substituenten und Indices folgende Bedeutung hat:
Ar 4-Chlorphenyl, 4-Fluorphenyl, 2,4-Dichlorphenyl, 2,4-Difluorphenyl, 4-Chlor-2-fluorphenyl, Fluorenyl, 4-Biphenylyl, 4-Phenoxyphenyl oder 4-Phenylthiophenyl bedeutet, wobei die drei letztgenannten Gruppen unsubstituiert oder in der 4'-Position mit Fluor, Chlor, Methyl, Methoxy oder der Trifluormethylgruppe substituiert sein können, $R^1$ und $R^2$ gleich oder verschieden sind und Wasserstoff, Fluor, Chlor, Trifluormethyl, Methyl oder Methoxy bedeuten.

5. Verfahren zur Herstellung von Verbindungen der Formel (II)

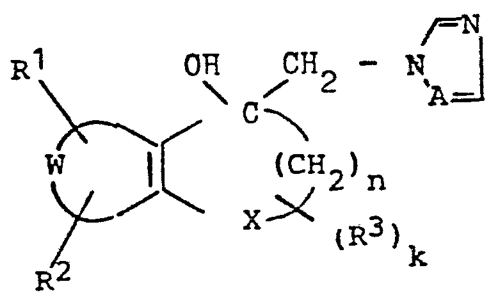

(II)

mit Einschränkung, daß (II) nicht die Verbindungen 1-Hydroxy-1-(azol-1-ylmethyl)-1,2,3,4-tetrahydronaphthalin bedeutet, wobei unter "Azol" 1H-Imidazol oder 1,2,4-Triazol zu verstehen ist, dadurch gekennzeichnet, daß man eine Verbindung der Formel (VI)

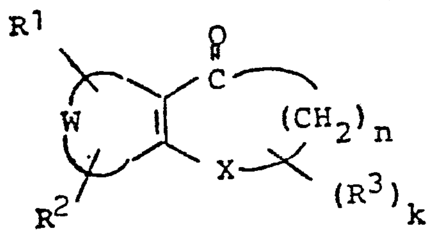

(VI)

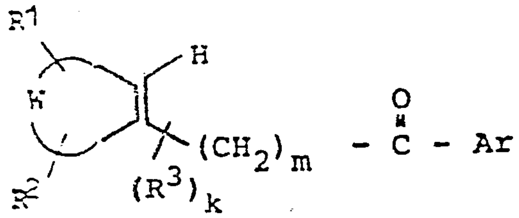
(X)

mit einem Schwefelylid der Formel (VII)

$$(CH_3)_2\underset{(O)_z}{S}CH_2 \qquad (VII)$$

worin z 0 oder 1 bedeutet,
zu einer Verbindung der Formel (XI) umsetzt

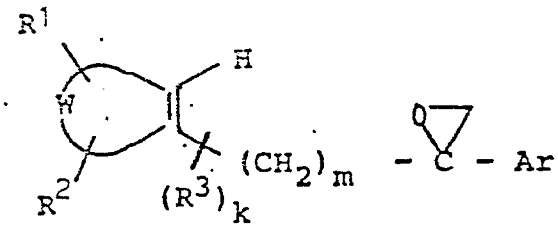
(XI)

und die Verbindung der Formel (XI) anschließend mit einer Verbindung der Formel (IX) nach Anspruch 5 zu einer Verbindung der Formel (IV) umsetzt.

7. Verfahren zur Herstellung von Verbindungen der Formel (X)

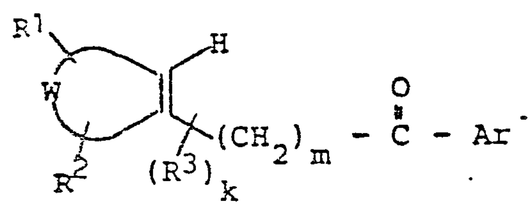
(X)

worin W, Ar, $R^1$, $R^2$, $R^3$ und k die in Anspruch 1 angegebenen Bedeutungen haben und m = 3 ist, dadurch gekennzeichnet, daß man eine Verbindung der Formel (XII)

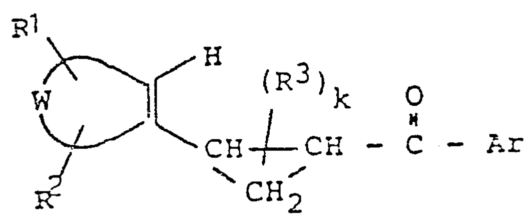
(XII)

in Gegenwart eines Metallkatalysators hydriert und so eine Verbindung der Formel (X) erhält.

mit einem Schwefelylid der Formel (VII)

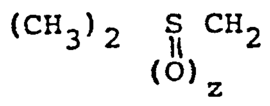

(VII)

worin z O oder 1 bedeutet,
zu einer Verbindung der Formel (VIII) umsetzt,

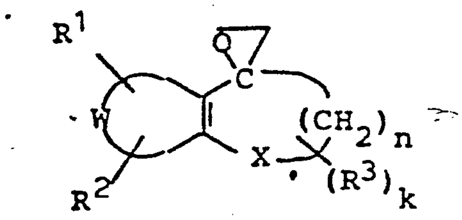

(VIII)

und anschließend die Verbindung der Formel (VIII) mit einer Verbindung der Formel (IX)

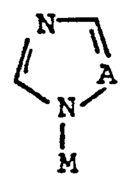

(IX)

worin A die in Anspruch 1 angegebenen Bedeutungen hat und M Wasserstoff, die Trimethylsilylgruppe oder ein Metalläquivalent bedeutet, zu einer Verbindung der Formel (II) umsetzt.

6. Verfahren zur Herstellung von Verbindungen der Formel (IV)

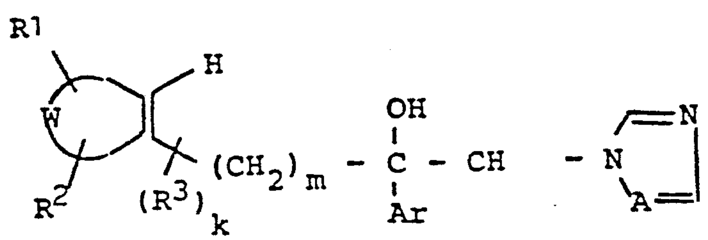

(IV)

worin A, Ar, W, $R^1$, $R^2$, $R^3$ und k die in Anspruch 1 angegebenen Bedeutungen haben und m 3 oder 4 bedeutet,
dadurch gekennzeichnet, daß man eine Verbindung der Formel (X)

8. Verwendung einer Verbindung I nach Ansprüchen 1,2,3 oder 4 zur Herstellung eines Medikaments.

9. Verwendung einer Verbindung I nach Ansprüchen 1,2,3 oder 4 zur Herstellung eines Medikaments zur Bekämpfung von Mykosen, Protozoen und grampositiver und gramnegativer Bakterien.

10. Verwendung einer Verbindung (I) nach Ansprüchen 1,2,3 oder 4 zur Herstellung eines Medikaments zur Behandlung und Verhütung von Konvulsionen.

11. Verfahren zur Bekämpfung oder Verhinderung eines Befalls von Kulturpflanzen durch phytopathogene Pilze, dadurch gekennzeichnet, daß man eine Verbindung der Formel (I) gemäß Ansprüchen 1,2,3 oder 4 auf die Pflanze bzw. auf durch Pilzbefall bedrohte Flächen, Pflanzen oder Saatgüter einwirken läßt.

12. Verwendung einer Verbindung II von Anspruch 1 zur Herstellung eines Medikaments zur Behandlung und Verhütung von Konvulsionen.

13. Verwendung einer Verbindung der Formel II von Anspruch 1 als Fungizid.